# EUROPEAN PATENT SPECIFICATION

(11) **EP 3 684 763 B1**
(45) Date of publication and mention of the grant of the patent: **17.11.2021**
(21) Application number: 18773414.0
(22) Date of filing: 18.09.2018
(51) Int. Cl.: C07D 401/04, A01N 43/54, A01N 43/84

(54) **PYRIMIDIN-4-CARBAMATE OR UREA DERIVATIVES AS HERBICIDES**
PYRIMIDIN-4-CARBAMAT ODER HARNSTOFF DERIVATIVE ALS HERBIZIDE
DÉRIVÉS DE PYRIMIDINE-4-CARBAMATE OU CARBAMIDE COMME HERBICIDES

(30) Priority: 22.09.2017 GB 201715324
(43) Date of publication of application: 29.07.2020
(73) Proprietor: Syngenta Participations AG, 4058 Basel (CH)
(72) Inventor: WAILES, Jeffrey, Steven, Bracknell Berkshire RG42 6EY (GB); BRIGGS, Emma, Bracknell Berkshire RG42 6EY (GB); CARTER, Neil, Brian, Bracknell Berkshire RG42 6EY (GB); MORRIS, Melloney, Bracknell Berkshire RG42 6EY (GB); TATE, Joseph, Andrew, Bracknell Berkshire RG42 6EY (GB)
(86) International application number: PCT/EP2018/075228
(87) International publication number: WO 2019/057722

(56) References cited:
- WO-A1-2017/162522
- JP-A- 2015 147 757

## Description

The present invention relates to herbicidally active pyridyl-/pyrimidyl-pyrimidine derivatives, as well as to processes and intermediates used for the preparation of such derivatives. The invention further extends to herbicidal compositions comprising such derivatives, as well as to the use of such compounds and compositions in controlling undesirable plant growth: in particular the use in controlling weeds, in crops of useful plants.

Certain pyrido-pyridine and pyrimidino-pyridine derivatives are known from JP2014-208631, where they are stated to have activity as insecticidal agents, and in particular miticidal agents.

The present invention is based on the finding that pyridyl-pyrimidine, and pyrimidylpyrimidine, derivatives of Formula (I) as defined herein, exhibit surprisingly good herbicidal activity. Thus, according to the present invention there is provided a compound of Formula (I) 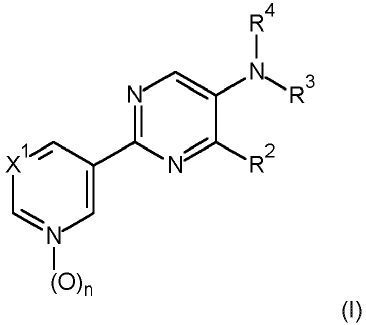 or a salt thereof, wherein,
X¹ is N or CR¹;
R¹ is selected from the group consisting of hydrogen, halogen, cyano, C₁-C₆alkyl, C₃-C₆cycloalkyl, C₂-C₆alkenyl, C₂-C₆alkynyl, C₁-C₆alkoxy, -C(O)OC₁-C₆alkyl, -S(O)ₚC₁-C₆alkyl, NR⁶R⁷, C₁-C₆haloalkoxy and C₁-C₆haloalkyl;
R² is selected from the group consisting of halogen, cyano, nitro, C₁-C₆alkyl, C₁-C₆haloalkyl, C₂-C₆alkenyl, C₂-C₆alkynyl, C₃-C₆cycloalkyl, -C(O)OC₁-C₆alkyl, -S(O)ₚ(C₁-C₆alkyl), C₁-C₆alkoxy, C₁-C₆haloalkoxy, phenyl, and benzyloxy;
R³ is -C(O)X²R¹²;
X² is O or NR¹⁰;
R⁴ is selected from the group consisting of hydrogen, C₁-C₆alkyl, C₁-C₆alkoxy, C₁-C₆haloalkyl, C₁-C₆haloalkoxy, C₃-C₆cycloalkyl, C₃-C₆alkenyl, C₃-C₆alkynyl, - C(O)R⁹ -(CR^{a}R^{b})_{q}R⁵, -C(O)X³R¹³; or,
when X² is O, R¹² is selected from the group consisting of C₁-C₆alkyl, CᵣalkoxyCₛalkyl, C₁-C₆haloalkyl, CᵣalkoxyCₛhaloalkyl, CᵣalkylthioCₛalkyl, C₂-C₆alkenyl, C₂-C₆alkynyl, and -(CR^{a}R^{b})_{q}R¹¹, or R⁴ and R¹² together with the heteroatoms to which they are joined form a 5-7 membered ring system optionally optionally containing 1 additional heteroatom selected from S, O and N, wherein when said additional heteroatom is sulphur it is in the form S(O)ₚ,
when X² is NR¹⁰, R¹² is selected from the group consisting of hydrogen, C₁-C₆alkyl, C₁-C₆alkoxy, C₁-C₆haloalkyl, C₁-C₆haloalkoxy, CᵣalkylthioCₛalkyl, C₂-C₆alkenyl, C₂-C₆alkynyl, and -(CR^{a}R^{b})_{q}R¹¹; or R¹⁰ and R¹² together with the nitrogen atom to which they are both joined, can form a 5-, 6-, or 7-memberered ring, optionally containing 1 to 3 additional heteroatoms each independently selected from O, N or S, wherein when said ring contains a ring sulphur, said ring sulphur is in the form S(O)ₚ; or R⁴ and R¹⁰ together with the atoms to which they are joined form a 5-7 membered ring system optionally comprising from 1 or 2 additional heteroatoms independently selected from S, O and N and wherein when said ring system contains a ring sulphur, said ring sulphur is in the form S(O)_{p;} or,
R¹⁰ is independently selected from the group consisting of hydrogen, C₁-C₆alkyl, C₃-C₆ cycloalkyl;
when R⁴ is -C(O)X³R¹³, X³ is O or NR¹⁴;
when X³ is O, R¹³ is selected from the group consisting of C₁-C₆alkyl, CᵣalkoxyCₛalkyl, C₁-C₆haloalkyl, CᵣalkoxyCₛhaloalkyl, CᵣalkylthioCₛalkyl, C₂-C₆alkenyl, C₂-C₆alkynyl, and -(CR^{a}R^{b})_{q}R¹¹, or R³ and R¹³ together with the heteroatoms to which they are joined form a 5-7 membered ring system optionally optionally containing 1 additional heteroatom selected from S, O and N, wherein when said additional heteroatom is sulphur it is in the form S(O)ₚ,
when X³ is NR¹⁴, R¹³ is selected from the group consisting of hydrogen, C₁-C₆alkyl, C₁-C₆alkoxy, C₁-C₆haloalkyl, C₁-C₆haloalkoxy, CᵣalkylthioCₛalkyl, C₂-C₆alkenyl, C₂-C₆alkynyl, and -(CR^{a}R^{b})_{q}R¹¹; or R¹⁴ and R¹³ together with the nitrogen atom to which they are both joined, can form a 5-, 6-, or 7-memberered ring, optionally containing 1 or 2 additional heteroatoms each independently selected from O, N or S, wherein when said ring contains a ring sulphur, said ring sulphur is in the form S(O)_{p;}
R^{a} is hydrogen or C₁-C₂ alkyl;
R^{b} is hydrogen or C₁-C₂ alkyl;
R⁵ is cyano, -C(O)OC₁-C₆alkyl, -C₃-C₆cycloalkyl, -aryl or ―heteroaryl wherein said aryl and heteroaryl are optionally substituted by 1 to 3 independent R⁸;
R⁶ and R⁷ are independently selected from the group consisting of hydrogen, C₁-C₆alkyl, and ―C(O)OC₁-C₄alkyl;
each R⁸ is independently selected from the group consisting of halogen, C₁-C₆alkyl and C₁-C₆alkoxy-, C₁-C₆ haloalkyl, C₁-C₆ haloalkoxy-, cyano and S(O)ₚ(C₁-C₆alkyl);
R⁹ is selected from the group consisting of hydrogen, C₁-C₆alkyl, C₁-C₆alkoxy, C₁-C₆haloalkyl, C₁-C₆haloalkoxy, C₂-C₆alkenyl, C₂-C₆alkynyl, and -(CR^{a}R^{b})_{q}R¹¹;
R¹¹ is cyano, -C₃-C₆cycloalkyl, or an ―aryl, -heteroaryl or -heterocyclyl ring, wherein said ring is optionally substituted by 1 to 3 independent R⁸, and wherein when said ring contains a ring sulphur, said ring sulphur is in the form S(O)ₚ;
n is 0 or 1;
p is 0, 1, or 2;
q is 0, 1, 2, 3, 4, 5 or 6;
r is 1, 2, 3, 4, or 5, s is 1, 2, 3, 4, or 5, and the sum of r+s is less than or equal to 6.

Compounds of formula (I) may exist as different geometric isomers, or in different tautomeric forms. This invention covers the use of all such isomers and tautomers, and mixtures thereof in all proportions, as well as isotopic forms such as deuterated compounds.

It may be the case that compounds of formula (I) may contain one or more asymmetric centers and may thus give rise to optical isomers and diastereomers. While shown without respect to stereochemistry, the present invention includes the use of all such optical isomers and diastereomers as well as the racemic and resolved, enantiomerically pure R and S stereoisomers and other mixtures of the R and S stereoisomers and agrochemically acceptable salts thereof.

Each alkyl moiety either alone or as part of a larger group (such as alkoxy, alkylthio, alkoxycarbonyl, alkylcarbonyl, alkylaminocarbonyl, or dialkylaminocarbonyl, *et al.*) may be straight-chained or branched. Typically, the alkyl is, for example, methyl, ethyl, n-propyl, isopropyl, n-butyl, sec-butyl, isobutyl, *tert-butyl,* n-pentyl, neopentyl, or n-hexyl. The alkyl groups are generally C₁-C₆ alkyl groups (except where already defined more narrowly), but are preferably C₁-C₄ alkyl or C₁-C₃ alkyl groups, and, more preferably, are C₁-C₂ alkyl groups (such as methyl).

Alkenyl and alkynyl moieties can be in the form of straight or branched chains, and the alkenyl moieties, where appropriate, can be of either the (E)- or (Z)-configuration. Alkenyl and alkynyl moieties can contain one or more double and/or triple bonds in any combination; but preferably contain only one double bond (for alkenyl) or only one triple bond (for alkynyl).

The alkenyl or alkynyl moieties are typically C₂-C₄ alkenyl or C₂-C₄ alkynyl, more specifically ethenyl (vinyl), prop-2-enyl, prop-3-enyl (allyl), ethynyl, prop-3-ynyl (propargyl), or prop-1-ynyl. Preferably, the term cycloalkyl refers to cyclopropyl, cyclobutyl, cyclopentyl or cyclohexyl.

In the context of the present specification the term "aryl" preferably means phenyl.

Heteroaryl groups and heteroaryl rings (either alone or as part of a larger group, such as heteroaryl-alkyl-) are ring systems containing at least one heteroatom and can be in mono- or bi-cyclic form. Typically "heteroaryl" is as used in the context of this invention includes furyl, thienyl, pyrrolyl, pyrazolyl, imidazolyl, triazolyl, oxazolyl, isoxazolyl, thiazolyl, isothiazolyl, oxadiazolyl, pyridyl, pyrimidinyl, pyridazinyl, pyrazinyl, and triazinyl rings, which may or may not be substituted as described herein.

The term "heterocyclyl" as used herein, encompasses ring systems containing at least one heteroatom and that are typically in monocyclic form. Preferably, heterocyclyl groups will contain up to two heteroatoms which will preferably be chosen from nitrogen, oxygen and sulfur. Where a heterocycle contains sulfur as a heteroatom it may be in oxidized form i.e. in the form ―S(O)ₚ- where p is an integer of 0, 1 or 2 as defined herein. Such heterocyclyl groups are preferably 3- to 8-membered, and more preferably 3- to 6-membered rings. Examples of heterocyclic groups include oxetanyl, thietanyl, and azetidinyl groups. Such heterocyclyl rings may or may not be substituted as described herein.

Halogen (or halo) encompasses fluorine, chlorine, bromine or iodine. The same correspondingly applies to halogen in the context of other definitions, such as haloalkyl or halophenyl.

Haloalkyl groups having a chain length of from 1 to 6 carbon atoms are, for example, fluoromethyl, difluoromethyl, trifluoromethyl, chloromethyl, dichloromethyl, trichloromethyl, 2,2,2-trifluoroethyl, 2-fluoroethyl, 2-chloroethyl, pentafluoroethyl, 1,1-difluoro-2,2,2-trichloroethyl, 2,2,3,3-tetrafluoroethyl and 2,2,2-trichloroethyl, heptafluoron-propyl and perfluoro-n-hexyl.

Alkoxy groups preferably have a chain length of from 1 to 6 carbon atoms. Alkoxy is, for example, methoxy, ethoxy, propoxy, isopropoxy, n-butoxy, isobutoxy, sec-butoxy or tert-butoxy or a pentyloxy or hexyloxy isomer, preferably methoxy and ethoxy. It should also be appreciated that two alkoxy substituents may be present on the same carbon atom.

Haloalkoxy is, for example, fluoromethoxy, difluoromethoxy, trifluoromethoxy, 2,2,2-trifluoroethoxy, 1,1,2,2-tetrafluoroethoxy, 2-fluoroethoxy, 2-chloroethoxy, 2,2-difluoroethoxy or 2,2,2-trichloroethoxy, preferably difluoromethoxy, 2-chloroethoxy or trifluoromethoxy.

C₁-C₆ alkyl-S- (alkylthio) is, for example, methylthio, ethylthio, propylthio, isopropylthio, n-butylthio, isobutylthio, sec-butylthio or tert-butylthio, preferably methylthio or ethylthio.

C₁-C₆ alkyl-S(O)- (alkylsulfinyl) is, for example, methylsulfinyl, ethylsulfinyl, propylsulfinyl, isopropylsulfinyl, n-butylsulfinyl, isobutylsulfinyl, sec-butylsulfinyl or tertbutylsulfinyl, preferably methylsulfinyl or ethylsulfinyl.

C₁-C₆ alkyl-S(O)₂- (alkylsulfonyl) is, for example, methylsulfonyl, ethylsulfonyl, propylsulfonyl, isopropylsulfonyl, n-butylsulfonyl, isobutylsulfonyl, sec-butylsulfonyl or tertbutylsulfonyl, preferably methylsulfonyl or ethylsulfonyl.

Compounds of formula (I) may form, and/or be used as, agronomically acceptable salts with amines (for example ammonia, dimethylamine and triethylamine), alkali metal and alkaline earth metal bases or quaternary ammonium bases. Among the alkali metal and alkaline earth metal hydroxides, oxides, alkoxides and hydrogen carbonates and carbonates used in salt formation, emphasis is to be given to the hydroxides, alkoxides, oxides and carbonates of lithium, sodium, potassium, magnesium and calcium, but especially those of sodium, magnesium and calcium. The corresponding trimethylsulfonium salt may also be used.

Compounds of formula (I) may also form (and /or be used as) agronomically acceptable salts with various organic and/or inorganic acids, for example, acetic, propionic, lactic, citric, tartaric, succinic, fumaric, maleic, malonic, mandelic, malic, phthalic, hydrochloric, hydrobromic, phosphoric, nitric, sulfuric, methanesulfonic, naphthalenesulfonic, benzenesulfonic, toluenesulfonic, camphorsulfonic, and similarly known acceptable acids, when the compound of formula (I) contains a basic moiety.

Where appropriate compounds of formula (I) may also be in the form of/used as an N-oxide.

Compounds of formula (I) may also be in the form of/used as hydrates which may be formed during the salt formation.

Preferred values of X¹, X², X³, R¹, R², R³, R⁴, R⁵, R⁶, R⁷, R⁸, R⁹, R¹⁰, R¹¹, R¹², R¹³, R¹⁴, R^{a}, R^{b}, n, p, q r and s are as set out below, and a compound of formula (I) according to the invention may comprise any combination of said values. The skilled person will appreciate that values for any specified set of embodiments may combined with values for any other set of embodiments where such combinations are not mutually exclusive.

The skilled man will also appreciate that the values or r and s in the definitions CᵣalkoxyCₛalkyl and CᵣalkoxyCₛhaloalkyl are such that the length of the carbon chain within the substituent does not exceed 6. Preferred values of r are 1, 2, or 3. Preferred values for s are 1, 2, or 3. In various embodiments r is 1, s is 1; or, r is 1, s is 2; or r is 1, s is 3; or r is 2, s is 1; r is 2, s is 2; or r is 2, s is 3; or r is 3, s is 1; or r is 3, s is 2, r is 3, s is 3. Particularly preferred substituents thus include methoxymethyl, and ethoxymethyl.

In one particular embodiment of the present invention, X¹ is N.

In another embodiment of the present invention, X¹ is CR¹ and R¹ is preferably selected from the group consisting of hydrogen, cyano, halogen, C₁-C₃alkyl, C₃-C₄alkynyl, C₁-C₃alkoxy, C₁-C₃haloalkyl, C₁-C₃haloalkoxy, NR⁶R⁷ and C₁-C₃thioalkyl. More preferably R¹ is selected from hydrogen, cyano, chloro, fluoro, methyl, propynyl, methoxy, trifluoromethyl, difluoromethoxy, NR⁶R⁷ as defined *infra,* and thiomethyl. More preferably still, R¹ is selected from the group consisting of hydrogen, cyano, fluoro, chloro, methoxy-, difluoromethyl, trifluoromethyl and NR⁶R⁷ as defined *infra.* Most preferably R¹ is fluoro.

Preferably R² is selected from the group consisting of halogen, cyano, C₁-C₆alkyl, C₁-C₆haloalkyl, C(O)OC₁-C₆alkyl, phenyl & benzyloxy. More preferably R² is chloro, cyano, methyl, trifluoromethyl, methoxy, -C(O)OCH₃, phenyl or benzyloxy.

In one set of embodiments R² is selected from the group consisting of halogen, cyano, nitro, C₁-C₆alkyl, C₁-C₆haloalkyl, C₂-C₆alkenyl, C₂-C₆alkynyl, C₃-C₆cycloalkyl, - C(O)OC₁-C₆alkyl, -S(O)ₚ(C₁-C₆alkyl), C₁-C₆alkoxy, C₁-C₆haloalkoxy, or benzyloxy and is preferably halogen, C₁-C₆alkyl, C₁-C₆haloalkyl, or benzyloxy, more preferably chloro, methyl, trifluoromethyl or benzyloxy, and more prefereably still methyl, trifluoromethyl, or benzyloxy.

Where X² is O (i.e. where R³ is ―C(O)OR¹²), R¹² is preferably selected from the group consisting of hydrogen, C₁-C₆alkyl, CᵣalkoxyCₛalkyl, C₁-C₆haloalkyl, CᵣalkoxyCₛhaloalkyl, CᵣalkylthioCₛalkyl, C₂-C₆alkenyl, C₂-C₆alkynyl, and -(CR^{a}R^{b})_{q}R¹¹. In such embodiments, R¹² is preferably C₁-C₄alkyl, C₁-C₄ haloalkyl, C₁-C₃alkoxyC₁-C₃alkyl, C₁-C₃alkylthioC₁-C₃alkyl, or CR^{a}R^{b}_{q}R¹¹, wherein q is 0, 1 or 2, R^{a} and R^{b} are each hydrogen, and R¹¹ is cyano, C₃-C₆cycloalkyl, a 5- or 6-membered heterocycle containing 1 or 2 heteroatoms independently selected from O and S wherein said S is in the form S(O)ₚ, or phenyl optionally substituted by 1-3 R⁸.

In one set of embodiments, R³ is -C(O)OC₁-C₆alkyl, and preferably selected from the group consisting of -C(O)O-methyl, -C(O)O-ethyl, -C(O)O-*n*-propyl, -C(O)O-*iso*propyl, -C(O)O-*iso*-butyl, -C(O)O-*sec*-butyl, and -C(O)O-*tert*-butyl.

Where X² is NR¹⁰ (i.e. where R³ is ―C(O)NR¹⁰R¹²) it is preferred that R¹⁰ is hydrogen or C₁-C₆alkyl (in particular methyl), or that it forms a 5-7 membered (preferably 5- or 6-membered) ring system optionally containing from 1 to 3 additional heteroatoms independently selected from S in the form of S(O)ₚ, O and N, in conjunction either with R⁴ and the atoms to which R¹⁰ and R⁴ are joined, or in conjunction with R¹² and the nitrogen atom to which R¹⁰ and R¹² are joined. In embodiments where R⁴ and R¹⁰ are joined, the skilled man will appreciate that the ring system may appear as a substituted ring system bearing a substituent on the nitrogen atom of group NR¹⁰, by virtue of substituent R¹². In these embodiments it is preferred that R¹² is hydrogen, or C₁-C₆ alkyl; preferably hydrogen or C₁-C₃ alkyl; and more preferably hydrogen or methyl. In one set of embodiments R¹⁰ and R⁴ together with the atoms to which they are joined form an optionally substituted (by R¹²) six-membered ring system, in particular a substituted hexahydropyrimidone.

In embodiments where R¹⁰ and R¹² together with the nitrogen atom to which they are joined form a ring system, it is preferred that said ring system is 5- or 6-membered. Where the ring system is 5-membered, it will preferably contain 0 or 1 additional heteroatom independently selected from O, N, or S in the form of S(O)ₚ. More preferably the 1 additional heteroatom will be S in the form of S(O)ₚ. Where the ring system is 6-membered, it will preferably contain 0 or 1 additional heteroatom independently selected from O, N, or S in the form of S(O)ₚ. More preferably the 1 additional heteroatom will be O or N, most preferably N. In one set of embodiments R¹⁰ and R¹² together with the N-atom to which they are joined form a morpholine ring.

Where R¹⁰ does not form a ring with either R⁴ or R¹², and is hydrogen or C₁-C₆alkyl (preferably hydrogen or methyl), it is preferred that R¹² is C₁-C₄alkyl, C₁-C₃alkoxy, - (CH₂)₃SCH₃, C₁-C₃haloalkyl, C₃-C₆alkynyl, or (CR^{a}R^{b})_{q}R¹¹. In such embodiments where R¹² is (CR^{a}R^{b})_{q}R¹¹, it is particularly preferred that q is 0 or 1. It is further preferred that R¹¹ in such embodiments is an optionally substituted ring system selected from the group consisting of C₃-C₆cycloalkyl, isoxazolyl, phenyl, pyridyl, pyrimidinyl, tetrahydropyranyl and morpholinyl, which, when substituted, is substituted by 1-3 independent R⁸.

Preferably, where R⁴ does not form a ring with either R¹⁰ or R¹², R⁴ is selected from the group consisting of hydrogen, methyl, ethyl, allyl, but-2-yn-1-yl, C(O)R⁹ where R⁹ is preferably C₁-C₆alkoxy, and -(CH₂)_{q}R⁵ wherein q is 1 and R⁵ is selected from the group consisting of c-propyl, -CO₂methyl, and phenyl optionally substituted by 1-2 groups R⁸, wherein each R⁸ is independently C₁-C₃alkyl or halogen (more preferably in such embodiments R⁸ is methyl or fluoro). In one embodiment where R⁴ is -(CH₂)_{q}R⁵, R⁴ is the group ―CH₂-2,4-difluorophenyl. In further embodiments where R⁴ is -(CH₂)_{q}R⁵, R⁴ is - ethyl-cyclopropyl, or ethyl-difluoro-benzyl. In particularly preferred embodiments R⁴ is selected from the group consisting of hydrogen, methyl, allyl, propoxycarbonyl, and butoxycarbonyl.

In an alternative embodiment of the present invention, R⁴ and R¹⁰ together with the atoms to which they are joined form a 5-7 membered ring system optionally containing from 1 to 3 heteroatoms independently selected from S, O and N, as described *supra.*

In yet a further alternative embodiment, R⁴ and R¹² together with the atoms to which they are joined form a a 5-7 membered ring system optionally containing 1 or 2 additional heteroatoms independently selected from S, O and N. Where an additional heteroatom is S, it will be in the form of S(O)ₚ. Preferably said ring system is 6-membered, more preferably said ring system is an oxazinanone ring.

In one particular embodiment R⁶ and R⁷ are both hydrogen. In another embodiment R⁶ is hydrogen and R⁷ is C₁-C₆alkyl (e.g., methyl or ethyl). In another embodiment, R⁶ and R⁷ are both C₁-C₆alkyl. In yet a further embodiment R⁶ is hydrogen and R7 is ―C(O)OC4alkyl (in particular ―C(O)O-tert-butyl).

Preferably R⁹ is C₁-C₆alkyl, preferably ethyl, propyl (in particular iso-propyl) or butyl (in particular *tert-butyl).*

Preferably R¹¹ is selected from the group consisting of C₃-C₆cycloalkyl, phenyl optionally substituted by 1-3 R⁸, a 5- or 6-membered unsubstituted heteroaryl or 5- or 6-membered unsubstituted heterocyclyl ring, and a 5- or 6-membered heteroaryl or 5- or 6-membered heterocyclyl ring, each substituted by 1-3 R⁸. When said phenyl, heterocyclyl or heteroaryl ring is substituted, it is preferably substituted by 1 or 2 R⁸.

Preferably each R⁸ is independently selected from halogen, C₁-C₃-alkyl or C₁-C₃haloalkyl. More preferably each R⁸ is independently selected from methyl, ethyl, chloro or fluoro, more preferably still methyl or chloro.

In one set of embodiments R¹¹ is cyclopropyl, cyclobutyl, cyclopentyl, cyclohexyl, phenyl, or phenyl substituted by 1-3 R⁸.

Table 1 below provides 22 specific examples of herbicidal compounds of Formula (I) for use according to the invention.

**Table 1 Specific examples of compounds of Formula (I-E) (which are compounds of formula (I) wherein n is 0 and X¹, R², R³, and R⁴ are given in the table) for use in the invention.**

| 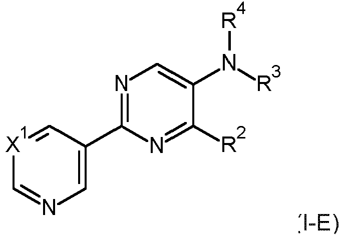 | | | | |
|---|---|---|---|---|
| **Compound** | **X¹** | **R²** | **R³** | **R⁴** |
| E1 | C-F | CH₃ | CO₂^{t}Bu | H |
| E2 | C-F | CH₃ | CO₂^{t}Bu | CH₃ |
| E3 | N | CH₃ | CO₂^{t}Bu | CH₃ |
| E4 | C-F | CF₃ | CO₂^{t}Bu | H |
| E5 | C-F | CF₃ | CO₂^{t}Bu | CH₃ |
| E6 | N | CH₃ | CO₂^{t}Bu | CH₂CH=CH₂ |
| E7 | N | CH₃ | CO₂^{t}Bu | H |
| E8 | C-F | CF₃ | 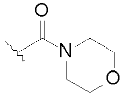 | H |
| E9 | C-F | CF₃ | 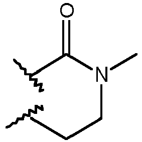 | |
| E10 | C-F | CF₃ | CO₂CH₂CH₂CH₃ | H |
| E11 | N | CF₃ | CO₂^{t}Bu | H |
| E12 | C-CN | CF₃ | CO₂^{t}Bu | H |
| E13 | C-F | CF₃ | C(O)N(CH₃)₂ | H |
| E14 | C-F | CF₃ | CO₂ⁱPr | H |
| E15 | C-F | CF₃ | CO₂CH₂CH(CH₃)₂ | H |
| E16 | C-NHCO₂^{t}Bu | CF₃ | CO₂^{t}Bu | H |
| E17 | C-F | CF₃ | 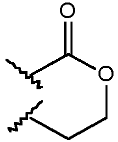 | |
| E18 | C-F | CF₃ | CO₂CH(CH₃)CH₂CH₃ | H |
| E19 | C-F | CF₃ | CO₂CH₂CH₃ | H |
| E20 | C-F | OCH₂Ph | CO₂^{t}Bu | H |
| E21 | C-F | CF₃ | CO₂CH₃ | H |
| E22 | C-F | CF₃ | CO₂ⁱPr | CO₂ⁱPr |

Compounds of Formula (I) may be prepared according to the following schemes, in which the substituents of X¹, X², X³, R¹, R², R³, R⁴, R⁵, R⁶, R⁷, R⁸, R⁹, R¹⁰, R¹¹, R¹², R¹³, R¹⁴, R^{a}, R^{b}, n, p, q r and s have (unless otherwise stated explicitly) the definitions described hereinbefore, using techniques known to the person skilled in the art of organic chemistry. General methods for the production of compounds of formula (I) are described below. The starting materials used for the preparation of the compounds of the invention may be purchased from the usual commercial suppliers or may be prepared by known methods. The starting materials as well as the intermediates may be purified before use in the next step by state of the art methodologies such as chromatography, crystallization, distillation and filtration.

Typical abbreviations used throughout are as follows:
app = apparent
BINAP = 2,2'-bis(diphenylphosphino)-1,1'-binaphthyl
br. = broad
^{t}Bu = tert-butyl
t-BuOH = *tert*-butanol
d = doublet
dd = double doublet
Dba = dibenzylideneacetone
DCM = dichloromethane
DMF = *N*, *N*-dimethylformamide
DMAP = 4-(dimethylamino)pyridine
DMSO = dimethylsulfoxide
DPPA = diphenylphosphoryl azide
Et₃N = triethylamine
Et₂O = diethyl ether
EtOAc = ethyl acetate
EtOH = ethanol
m = multiplet
mCPBA = *meta*-chloro-perbenzoic acid
Me = methyl
MeOH = methanol
Ms = mesylate
NaOEt = sodium ethoxide
NaOMe = sodium methoxide
NaSMe = sodium thiomethoxide
Ph = phenyl
ⁱPr = iso-propyl
q = quartet
RT or rt = room temperature
s = singlet
t = triplet
Tf = triflate
TFA = trifluoroacetic acid
THF = tetrahydrofuran
TMS = tetramethylsilane
tr = retention time

### Processes for preparation of compounds, e.g. compounds of formula (I)

Processes for preparation of compounds, e.g. a compound of formula (I) (which optionally can be an agrochemically acceptable salt thereof), are now described, and form further aspects of the present invention.

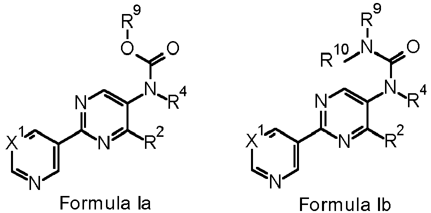

Compounds of Formula Ia are compounds of Formula (I) where X² = O, compounds of Formula Ib are compounds of Formula I where X² = NR¹⁰

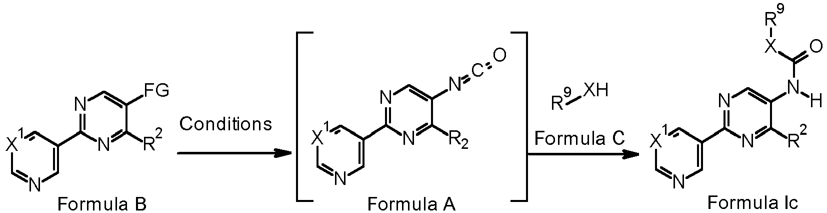

A compound of Formula Ic (a compound of Formula (I) where R³ is hydrogen) may be prepared from a compound of Formula A by reaction with a compound of Formula C, optionally in the presence of a suitable base and in a suitable solvent. The compound of formula A (isocyanate) may be prepared *in situ* from a suitable compound of Formula B (where FG represents a suitable functional group for example a carboxylic acid group) via a Curtius rearrangement with a suitable reagent such as diphenyl phosphoryl azide. Other methods for generating an isocyanate *in situ* are known in the literature. Alternatively the isocyanate can be prepared and isolated before reaction with a compound of Formula C, again methods for such a procedure are known in the literature. Compounds of Formula C are commercially available or can be prepared by methods well known in the literature.

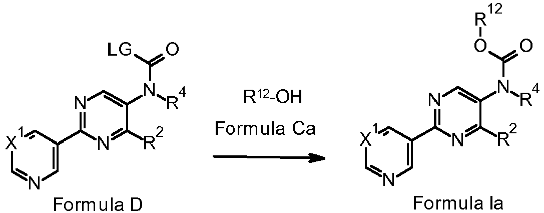

A compound of Formula Ia may be prepared from a compound of Formula D (where LG is a suitable leaving group, such as Cl or p-NO₂-phenol by reaction with a compound of Formula Ca (a compound of Formula C where X = O), optionally in the presence of a suitable base in a suitable solvent. Suitable bases include sodium hydride, *N*-ethyl-*N*,*N*-diisopropylamine, pyridine, 4-dimethylaminopyridine or triethylamine. Suitable solvents may include CH₃CN, THFor CH₂Cl₂.

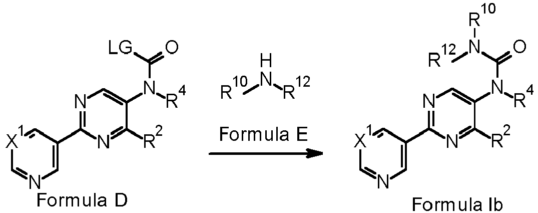

A compound of Formula Ib may be prepared from a compound of Formula D (where LG is a suitable leaving group, such as Cl or p-NO₂-phenol by reaction with a compound of Formula E, optionally in the presence of a suitable base in a suitable solvent. Suitable bases include sodium hydride, *N*-ethyl-*N*,*N*-diisopropylamine, pyridine, 4-dimethylaminopyridine or triethylamine. Suitable solvents may include CH₃CN, THF, DMSO or CH₂Cl₂.

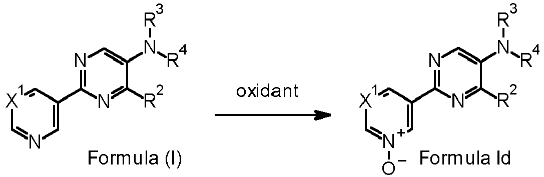

A compound of Formula Id (a compound of Formula (I) where n = 1) may be prepared from a compound of Formula (I) (where n = 0) *via* reaction with a suitable oxidant in a suitable solvent. Suitable oxidants may include 3-chloroperbenzoic acid. Suitable solvents may include DCM.

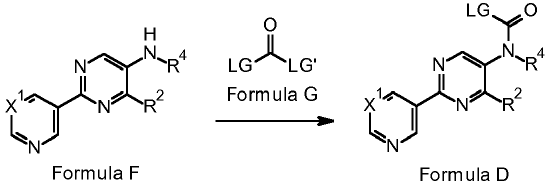

A compound of Formula D may be prepared from a compound of Formula F by reaction with a compound of Formula G (where LG' is a suitable leaving group such as CI), optionally in the presence of a suitable base and in a suitable solvent. Suitable bases may include pyridine. Suitable solvents may include CH₂Cl₂.

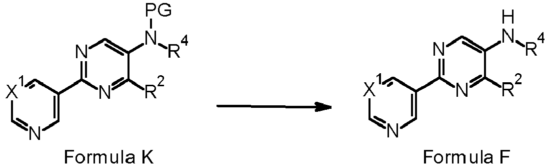

A compound of Formula F may be prepared from a compound of Formula K (where PG is a suitable protecting group such as *tert*-butoxycarbonyl) via a deprotection reaction using a suitable reagent in a suitable solvent. Suitable reagents for removal of a *tert-*butoxycarbonyl group include trifluoroacetic acid or hydrochloric acid. Suitable solvents may include CH₂Cl₂ or EtOAc.

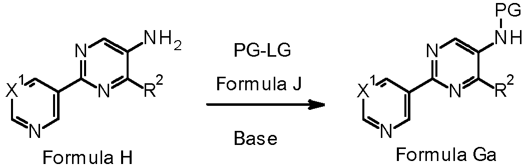

A compound of Formula Ga (a compound of Formula G where R⁴ is H and where PG is a suitable protecting group such as *tert*-butoxycarbonyl) may be prepared from a compound of Formula H via reaction with a compound of Formula J (where LG is a suitable leaving group, such as O^{t}Bu) optionally in the presence of a suitable base and in a suitable solvent. A suitable compound of Formula J may include di-*tert*-butyldicarbonate. Suitable bases may include lithium hexamethyldisilazide. Suitable solvents may include THF. Compounds of Formula J are commercially available or can be prepared by methods well known in the literature.

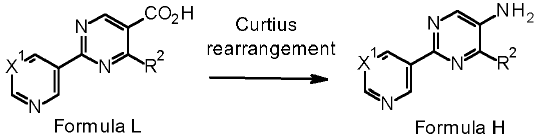

In an alternative approach, a compound of Formula H may be prepared from a compound of Formula L via a Curtius rearrangement using a suitable reagent in a suitable solvent. Suitable reagents include DPPA and suitable solvents include DMF or toluene.

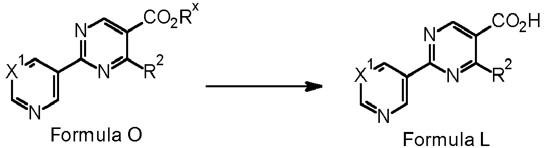

A compound of Formula L may be prepared from a compound of Formula O (where R^{x} is C₁₋₆ alkyl) via a hydrolysis reaction in the presence of a suitable reagent in a suitable solvent. Suitable reagents include NaOH, LiOH or KOH. Suitable solvents include H₂O, THF, MeOH or EtOH or mixtures thereof.

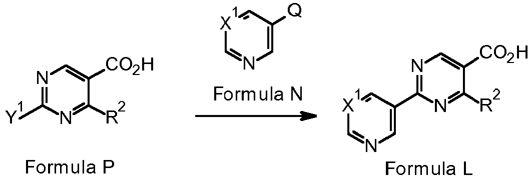

In an alternative approach, a compound of Formula L may be prepared from a compound of Formula P (where Y¹ is a suitable halogen, such as Cl or Br) via a cross-coupling reaction with a compound of Formula N (where Q is a suitable coupling group, such as ―B(OH)₂ or ―B(OR)₂ or ―SnR₃) in the presence of a suitable catalyst, optionally in the presence of a suitable base and in a suitable solvent. Suitable catalysts may include Pd(PPh₃)₄ or [1,1'-bis(diphenylphosphino)ferrocene]dichloropalladium(II). Suitable bases may include K₂CO₃, Na₂CO₃, Cs₂CO₃, K₃PO₄ or CsF. Suitable solvents may include ethylene glycol dimethyl ether, acetonitrile, DMF, ethanol, 1,4-dioxane, tetrahydrofuran and/or water. Compounds of Formula N are commercially available or can be prepared by methods well known in the literature.

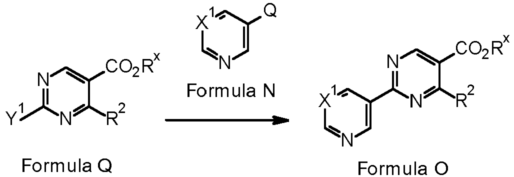

A compound of Formula O may be prepared from a compound of Formula Q (where Y¹ is a suitable halogen, such as Cl or Br) via a cross-coupling reaction with a compound of Formula N (where Q is a suitable coupling group, such as ―B(OH)₂ or ― B(OR)₂ or ―SnR₃) in the presence of a suitable catalyst, optionally in the presence of a suitable base and in a suitable solvent. Suitable catalysts may include Pd(PPh₃)₄ or [1,1'-bis(diphenylphosphino)ferrocene]dichloropalladium(II). Suitable bases may include K₂CO₃, Na₂CO₃, Cs₂CO₃, K₃PO₄ or CsF. Suitable solvents may include ethylene glycol dimethyl ether, acetonitrile, DMF, ethanol, 1,4-dioxane, tetrahydrofuran and/or water. Compounds of Formula N are commercially available or can be prepared by methods well known in the literature.

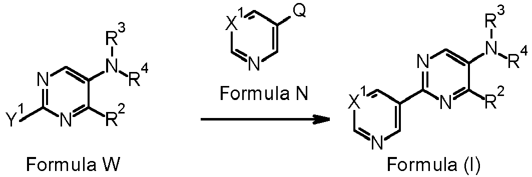

In a further alternative approach, a compound of Formula (I) may be prepared from a compound of Formula W (where Y¹ is a suitable halogen, such as Cl, Br or I or a suitable pseudohalogen, such as OTf) via a cross-coupling reaction with a compound of Formula N (where Q is a suitable coupling group, such as ―B(OH)₂ or ―B(OR)₂ or ―SnR₃) in the presence of a suitable catalyst, optionally in the presence of a suitable base and in a suitable solvent. Suitable catalysts may include Pd(PPh₃)₄, Pd₂Cl₂(PPh₃)₂, [1,1'-bis(diphenylphosphino)ferrocene]dichloropalladium(II). Suitable bases may include K₂CO₃ or CsF. Suitable solvents may include ethylene glycol dimethyl ether, acetonitrile, DMF, ethanol, 1,4-dioxane and/or water. Compounds of Formula N are commercially available or can be prepared by methods well known in the literature.

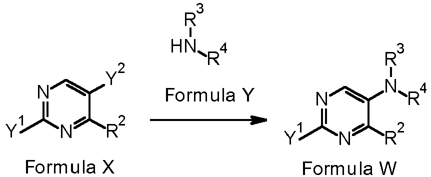

A compound of Formula W may be prepared from a compound of Formula X (where Y² is a suitable halogen, such as Br or I) via reaction with a compound of Formula Y, optionally in the presence of a suitable catalyst and optionally in the presence of a suitable base and in a suitable solvent. Suitable catalyst/ligand systems include Pd₂dba₃/BINAP. Suitable bases include NaO^{t}Bu. Suitable solvents include toluene or tetrahydrofuran Compounds of Formula Y and of Formula X are commercially available or can be prepared by methods well known in the literature.

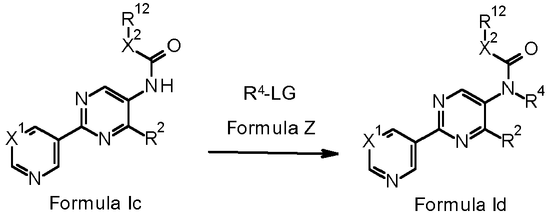

In a further alternative approach a compound of Formula Id (a compound of Formula (I) where R⁴ is not hydrogen) may be prepared from a compound of Formula Ic (a compound of Formula I where R⁴ is hydrogen) via an alkylation reaction with a compound of Formula Z in the presence of a suitable base and in a suitable solvent. Suitable bases may include sodium hydride or sodium hexamethyldisilazide. Suitable solvents may include THF and/or DMF. Compounds of Formula Z are commercially available or may be prepared by methods well known in the literature.

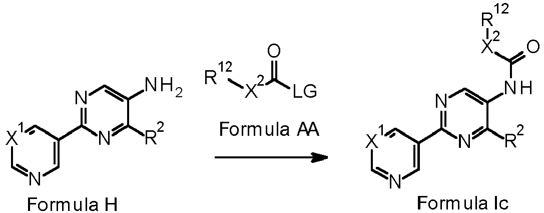

A compound of Formula Ic may be prepared from a compound of Formula H via an acylation reaction with a compound of Formula AA (where LG = a suitable leaving group such as Cl) optionally in the presence of a suitable base and in a suitable solvent. Suitable bases may include NaOH or pyridine. Suitable solvents may include acetone, THF, EtOAc and/or water. Compounds of Formula AA are commercially available or may be prepared by methods well known in the literature.

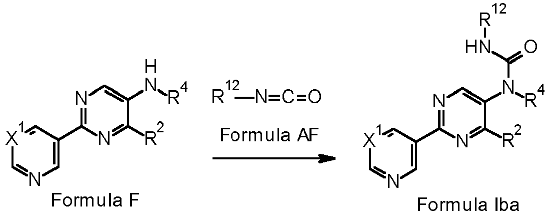

In an alternative approach a compound of Formula Iba (a compound of Formula Ib where R¹⁰ is hydrogen) may be prepared from a compound of Formula F *via* reaction with a compound of Formula AF optionally in the presence of a suitable base and in a suitable solvent. Suitable bases may include triethylamine or pyridine. Suitable solvents may include dichloromethane, toluene or tetrahydrofuran. Compounds of Formula AF are commercially available or may be prepared by methods well known in the literature.

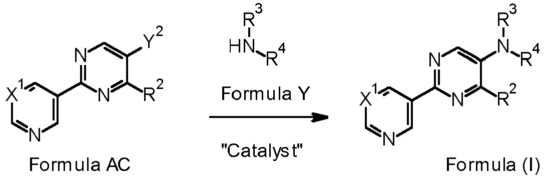

In a yet further alternative approach, a compound of Formula (I) may be prepared from a compound of Formula AC (where Y² is a suitable halogen, such as Cl, Br or I or a suitable pseudohalogen, such as OTf) *via* cross-coupling with a compound of Formula Y in the presence of a suitable catalyst/ligand, optionally in the presence of a suitable base and in a suitable solvent. Suitable catalyst/ligand combinations may include tris-(dibenzylideneacetone)dipalladium/9,9-dimethyl-4,5-bis(diphenyl-phosphino)xanthene (XantPhos), Pd(OAc)₂/2-(dicyclohexylphosphino)-2',4',6'-tri-i-propyl-1,1'-biphenyl or copper(l) iodide/1,2-diaminocyclohexane. Suitable bases include Cs₂CO₃ or K₃PO₄. Suitable solvents include 1,4-dioxane. Compounds of Formula Y are commercially available or may be prepared by methods well known in the literature.

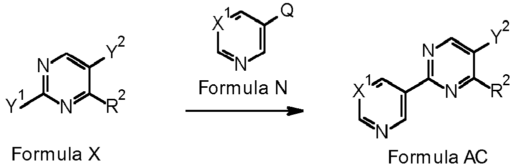

A compound of Formula AC may be prepared from a compound of Formula X (where Y¹ is a suitable halogen, such as Cl or Br) *via* a cross-coupling reaction with a compound of Formula N (where Q is a suitable coupling group, such as ―B(OH)₂ or ― B(OR)₂ or ―SnR₃) in the presence of a suitable catalyst, optionally in the presence of a suitable base and in a suitable solvent. Suitable catalysts may include Pd(PPh₃)₄, Pd₂Cl₂(PPh₃)₂ or [1,1'-bis(diphenylphosphino)ferrocene]dichloropalladium(II). Suitable bases may include K₂CO₃ or CsF. Suitable solvents may include ethylene glycol dimethyl ether, acetonitrile, DMF, ethanol, 1,4-dioxane and/or water. Compounds of Formula AD and of Formula N are commercially available or can be prepared by methods well known in the literature.

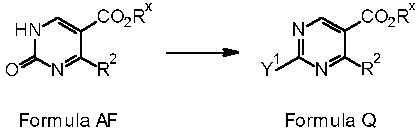

A compound of Formula Q (where Y¹ is a suitable halogen, such as Br or Cl) may be prepared from a compound of Formula AF via a halogenation reaction using a suitable reagent, optionally in a suitable solvent. Suitable reagents may include POCl₃. Suitable solvents may include DCM or DCE. Compounds of Formula AF are commercially available or may be prepared by methods well known in the literature. 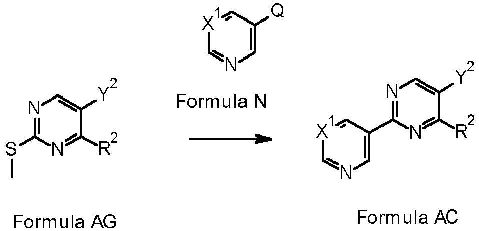 In an alternative approach, a compound of Formula AC may be prepared from a compound of Formula Q via a cross-coupling reaction (known as a Liebeskind-Srogl coupling) with a compound of Formula AG (where Q is a suitable coupling group, such as ―B(OH)₂) in the presence of a suitable catalyst, a suitable ligand, a suitable co-catalyst and in a suitable solvent. Suitable catalysts may include tris(dibenzylideneacetone)dipalladium(0), suitable ligands may include tris(2-furyl)phosphane, suitable co-catalysts may include copper(l) 3-methylsalicylate and suitable solvents may include THF. Compounds of Formula N are commercially available or can be prepared by methods well known in the literature.

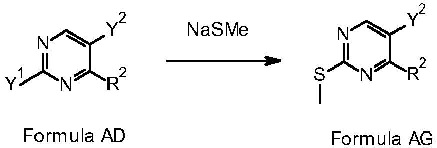

A compound of Formula AG may be prepared from a compound of Formula AD (where Y¹ is a suitable halogen, such as Cl) *via* a displacement reaction with NaSMe in a suitable solvent. Suitable solvents may include MeOH.

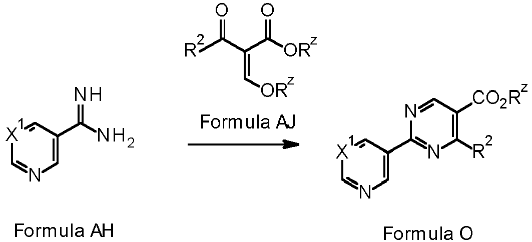

In a yet further alternative approach, a compound of Formula O may be prepared from a compound of Formula AH *via* a cyclisation reaction with a compound of Formula AJ (where R^{z} = C₁-C₃ alkyl) in the presence of a suitable base and in a suitable solvent. Suitable bases may include NaOEt. Suitable solvents may include EtOH. Compounds of Formula AH and of Formula AJ are commercially available or may be prepared by methods well known in the literature.

The compounds of Formula (I) as described herein may be used as herbicides by themselves, but they are generally formulated into herbicidal compositions using formulation adjuvants, such as carriers, solvents and surface-active agents (SFAs). Thus, the present invention further provides a herbicidal composition comprising a herbicidal compound as described herein and an agriculturally acceptable formulation adjuvant. The composition can be in the form of concentrates which are diluted prior to use, although ready-to-use compositions can also be made. The final dilution is usually made with water, but can be made instead of, or in addition to, water, with, for example, liquid fertilisers, micronutrients, biological organisms, oil or solvents.

Such herbicidal compositions generally comprise from 0.1 to 99 % by weight, especially from 0.1 to 95 % by weight of compounds of Formula (I) and from 1 to 99.9 % by weight of a formulation adjuvant, which preferably includes from 0 to 25 % by weight of a surface-active substance.

The compositions can be chosen from a number of formulation types, many of which are known from the Manual on Development and Use of FAO Specifications for Plant Protection Products, 5th Edition, 1999. These include dustable powders (DP), soluble powders (SP), water soluble granules (SG), water dispersible granules (WG), wettable powders (WP), granules (GR) (slow or fast release), soluble concentrates (SL), oil miscible liquids (OL), ultra low volume liquids (UL), emulsifiable concentrates (EC), dispersible concentrates (DC), emulsions (both oil in water (EW) and water in oil (EO)), micro-emulsions (ME), suspension concentrates (SC), aerosols, capsule suspensions (CS) and seed treatment formulations. The formulation type chosen in any instance will depend upon the particular purpose envisaged and the physical, chemical and biological properties of the compound of Formula (I).

Dustable powders (DP) may be prepared by mixing a compound of Formula (I) with one or more solid diluents (for example natural clays, kaolin, pyrophyllite, bentonite, alumina, montmorillonite, kieselguhr, chalk, diatomaceous earths, calcium phosphates, calcium and magnesium carbonates, sulphur, lime, flours, talc and other organic and inorganic solid carriers) and mechanically grinding the mixture to a fine powder.

Soluble powders (SP) may be prepared by mixing a compound of Formula (I) with one or more water-soluble inorganic salts (such as sodium bicarbonate, sodium carbonate or magnesium sulphate) or one or more water-soluble organic solids (such as a polysaccharide) and, optionally, one or more wetting agents, one or more dispersing agents or a mixture of said agents to improve water dispersibility/solubility. The mixture is then ground to a fine powder. Similar compositions may also be granulated to form water soluble granules (SG).

Wettable powders (WP) may be prepared by mixing a compound of Formula (I) with one or more solid diluents or carriers, one or more wetting agents and, preferably, one or more dispersing agents and, optionally, one or more suspending agents to facilitate the dispersion in liquids. The mixture is then ground to a fine powder. Similar compositions may also be granulated to form water dispersible granules (WG).

Granules (GR) may be formed either by granulating a mixture of a compound of Formula (I) and one or more powdered solid diluents or carriers, or from pre-formed blank granules by absorbing a compound of Formula (I) (or a solution thereof, in a suitable agent) in a porous granular material (such as pumice, attapulgite clays, fuller's earth, kieselguhr, diatomaceous earths or ground corn cobs) or by adsorbing a compound of Formula (I) (or a solution thereof, in a suitable agent) on to a hard core material (such as sands, silicates, mineral carbonates, sulphates or phosphates) and drying if necessary. Agents which are commonly used to aid absorption or adsorption include solvents (such as aliphatic and aromatic petroleum solvents, alcohols, ethers, ketones and esters) and sticking agents (such as polyvinyl acetates, polyvinyl alcohols, dextrins, sugars and vegetable oils). One or more other additives may also be included in granules (for example an emulsifying agent, wetting agent or dispersing agent).

Dispersible Concentrates (DC) may be prepared by dissolving a compound of Formula (I) in water or an organic solvent, such as a ketone, alcohol or glycol ether. These solutions may contain a surface active agent (for example to improve water dilution or prevent crystallisation in a spray tank).

Emulsifiable concentrates (EC) or oil-in-water emulsions (EW) may be prepared by dissolving a compound of Formula (I) in an organic solvent (optionally containing one or more wetting agents, one or more emulsifying agents or a mixture of said agents). Suitable organic solvents for use in ECs include aromatic hydrocarbons (such as alkylbenzenes or alkylnaphthalenes, exemplified by SOLVESSO 100, SOLVESSO 150 and SOLVESSO 200; SOLVESSO is a Registered Trade Mark), ketones (such as cyclohexanone or methylcyclohexanone) and alcohols (such as benzyl alcohol, furfuryl alcohol or butanol), N-alkylpyrrolidones (such as N-methylpyrrolidone or N-octylpyrrolidone), dimethyl amides of fatty acids (such as C₈-C₁₀ fatty acid dimethylamide) and chlorinated hydrocarbons. An EC product may spontaneously emulsify on addition to water, to produce an emulsion with sufficient stability to allow spray application through appropriate equipment.

Preparation of an EW involves obtaining a compound of Formula (I) either as a liquid (if it is not a liquid at room temperature, it may be melted at a reasonable temperature, typically below 70°C) or in solution (by dissolving it in an appropriate solvent) and then emulsifying the resultant liquid or solution into water containing one or more SFAs, under high shear, to produce an emulsion. Suitable solvents for use in EWs include vegetable oils, chlorinated hydrocarbons (such as chlorobenzenes), aromatic solvents (such as alkylbenzenes or alkylnaphthalenes) and other appropriate organic solvents which have a low solubility in water.

Microemulsions (ME) may be prepared by mixing water with a blend of one or more solvents with one or more SFAs, to produce spontaneously a thermodynamically stable isotropic liquid formulation. A compound of Formula (I) is present initially in either the water or the solvent/SFA blend. Suitable solvents for use in MEs include those hereinbefore described for use in in ECs or in EWs. An ME may be either an oil-in-water or a water-in-oil system (which system is present may be determined by conductivity measurements) and may be suitable for mixing water-soluble and oil-soluble pesticides in the same formulation. An ME is suitable for dilution into water, either remaining as a microemulsion or forming a conventional oil-in-water emulsion.

Suspension concentrates (SC) may comprise aqueous or non-aqueous suspensions of finely divided insoluble solid particles of a compound of Formula (I). SCs may be prepared by ball or bead milling the solid compound of Formula (I) in a suitable medium, optionally with one or more dispersing agents, to produce a fine particle suspension of the compound. One or more wetting agents may be included in the composition and a suspending agent may be included to reduce the rate at which the particles settle. Alternatively, a compound of Formula (I) may be dry milled and added to water, containing agents hereinbefore described, to produce the desired end product.

Aerosol formulations comprise a compound of Formula (I) and a suitable propellant (for example n-butane). A compound of Formula (I) may also be dissolved or dispersed in a suitable medium (for example water or a water miscible liquid, such as *n-*propanol) to provide compositions for use in non-pressurised, hand-actuated spray pumps.

Capsule suspensions (CS) may be prepared in a manner similar to the preparation of EW formulations but with an additional polymerisation stage such that an aqueous dispersion of oil droplets is obtained, in which each oil droplet is encapsulated by a polymeric shell and contains a compound of Formula (I) and, optionally, a carrier or diluent therefor. The polymeric shell may be produced by either an interfacial polycondensation reaction or by a coacervation procedure. The compositions may provide for controlled release of the compound of Formula (I) and they may be used for seed treatment. A compound of Formula (I) may also be formulated in a biodegradable polymeric matrix to provide a slow, controlled release of the compound.

The composition may include one or more additives to improve the biological performance of the composition, for example by improving wetting, retention or distribution on surfaces; resistance to rain on treated surfaces; or uptake or mobility of a compound of Formula (I). Such additives include surface active agents (SFAs), spray additives based on oils, for example certain mineral oils or natural plant oils (such as soy bean and rape seed oil), and blends of these with other bio-enhancing adjuvants (ingredients which may aid or modify the action of a compound of Formula (I)).

Wetting agents, dispersing agents and emulsifying agents may be SFAs of the cationic, anionic, amphoteric or non-ionic type.

Suitable SFAs of the cationic type include quaternary ammonium compounds (for example cetyltrimethyl ammonium bromide), imidazolines and amine salts.

Suitable anionic SFAs include alkali metals salts of fatty acids, salts of aliphatic monoesters of sulphuric acid (for example sodium lauryl sulphate), salts of sulphonated aromatic compounds (for example sodium dodecylbenzenesulphonate, calcium dodecylbenzenesulphonate, butylnaphthalene sulphonate and mixtures of sodium di*iso*propyl- and tri-*iso*propyl-naphthalene sulphonates), ether sulphates, alcohol ether sulphates (for example sodium laureth-3-sulphate), ether carboxylates (for example sodium laureth-3-carboxylate), phosphate esters (products from the reaction between one or more fatty alcohols and phosphoric acid (predominately mono-esters) or phosphorus pentoxide (predominately di-esters), for example the reaction between lauryl alcohol and tetraphosphoric acid; additionally these products may be ethoxylated), sulphosuccinamates, paraffin or olefine sulphonates, taurates and lignosulphonates.

Suitable SFAs of the amphoteric type include betaines, propionates and glycinates.

Suitable SFAs of the non-ionic type include condensation products of alkylene oxides, such as ethylene oxide, propylene oxide, butylene oxide or mixtures thereof, with fatty alcohols (such as oleyl alcohol or cetyl alcohol) or with alkylphenols (such as octylphenol, nonylphenol or octylcresol); partial esters derived from long chain fatty acids or hexitol anhydrides; condensation products of said partial esters with ethylene oxide; block polymers (comprising ethylene oxide and propylene oxide); alkanolamides; simple esters (for example fatty acid polyethylene glycol esters); amine oxides (for example lauryl dimethyl amine oxide); and lecithins.

Suitable suspending agents include hydrophilic colloids (such as polysaccharides, polyvinylpyrrolidone or sodium carboxymethylcellulose) and swelling clays (such as bentonite or attapulgite).

Herbicidal compositions as described herein may further comprise at least one additional pesticide. For example, the compounds of formula (I) can also be used in combination with other herbicides or plant growth regulators. In a preferred embodiment the additional pesticide is a herbicide and/or herbicide safener. Examples of such mixtures are, in which 'I' represents a compound of Formula (I), I + acetochlor, I + acifluorfen, I + acifluorfen-sodium, I + aclonifen, I + acrolein, I + alachlor, I + alloxydim, I + ametryn, I + amicarbazone, I + amidosulfuron, I + aminopyralid, I + amitrole, I + anilofos, I + asulam, I + atrazine, I + azafenidin, I + azimsulfuron, I + BCPC, I + beflubutamid, I + benazolin, I + bencarbazone, I + benfluralin, I + benfuresate, I + bensulfuron, I + bensulfuron-methyl, I + bensulide, I + bentazone, I + benzfendizone, I + benzobicyclon, I + benzofenap, I + bicyclopyrone, I + bifenox, I + bilanafos, I + bispyribac, I + bispyribac-sodium, I + borax, I + bromacil, I + bromobutide, I + bromoxynil, I + butachlor, I + butamifos, I + butralin, I + butroxydim, I + butylate, I + cacodylic acid, I + calcium chlorate, I + cafenstrole, I + carbetamide, I + carfentrazone, I + carfentrazone-ethyl, I + chlorflurenol, I + chlorflurenol-methyl, I + chloridazon, I + chlorimuron, I + chlorimuron-ethyl, I + chloroacetic acid, I + chlorotoluron, I + chlorpropham, I + chlorsulfuron, I + chlorthal, I + chlorthal-dimethyl, I + cinidon-ethyl, I + cinmethylin, I + cinosulfuron, I + cisanilide, I + clethodim, I + clodinafop, I + clodinafop-propargyl, I + clomazone, I + clomeprop, I + clopyralid, I + cloransulam, I + cloransulam-methyl, I + cyanazine, I + cycloate, I + cyclosulfamuron, I + cycloxydim, I + cyhalofop, I + cyhalofop-butyl,, I + 2,4-D, I + daimuron, I + dalapon, I + dazomet, I + 2,4-DB, I + I + desmedipham, I + dicamba, I + dichlobenil, I + dichlorprop, I + dichlorprop-P, I + diclofop, I + diclofop-methyl, I + diclosulam, I + difenzoquat, I + difenzoquat metilsulfate, I + diflufenican, I + diflufenzopyr, I + dimefuron, I + dimepiperate, I + dimethachlor, I + dimethametryn, I + dimethenamid, I + dimethenamid-P, I + dimethipin, I + dimethylarsinic acid, I + dinitramine, I + dinoterb, I + diphenamid, I + dipropetryn, I + diquat, I + diquat dibromide, I + dithiopyr, I + diuron, I + endothal, I + EPTC, I + esprocarb, I + ethalfluralin, I + ethametsulfuron, I + ethametsulfuron-methyl, I + ethephon, I + ethofumesate, I + ethoxyfen, I + ethoxysulfuron, I + etobenzanid, I + fenoxaprop-P, I + fenoxaprop-P-ethyl, I + fentrazamide, I + ferrous sulfate, I + flamprop-M, I + flazasulfuron, I + florasulam, I + fluazifop, I + fluazifop-butyl, I + fluazifop-P, I + fluazifop-P-butyl, I + fluazolate, I + flucarbazone, I + flucarbazone-sodium, I + flucetosulfuron, I + fluchloralin, I + flufenacet, I + flufenpyr, I + flufenpyr-ethyl, I + flumetralin, I + flumetsulam, I + flumiclorac, I + flumiclorac-pentyl, I + flumioxazin, I + flumipropin, I + fluometuron, I + fluoroglycofen, I + fluoroglycofen-ethyl, I + fluoxaprop, I + flupoxam, I + flupropacil, I + flupropanate, I + flupyrsulfuron, I + flupyrsulfuron-methyl-sodium, I + flurenol, I + fluridone, I + flurochloridone, I + fluroxypyr, I + flurtamone, I + fluthiacet, I + fluthiacet-methyl, I + fomesafen, I + foramsulfuron, I + fosamine, I + glufosinate, I + glufosinate-ammonium, I + glyphosate, I + halauxifen, I + halosulfuron, I + halosulfuron-methyl, I + haloxyfop, I + haloxyfop-P, I + hexazinone, I + imazamethabenz, I + imazamethabenz-methyl, I + imazamox, I + imazapic, I + imazapyr, I + imazaquin, I + imazethapyr, I + imazosulfuron, I + indanofan, I + indaziflam, I + iodomethane, I + iodosulfuron, I + iodosulfuron-methyl-sodium, I + ioxynil, I + isoproturon, I + isouron, I + isoxaben, I + isoxachlortole, I + isoxaflutole, I + isoxapyrifop, I + karbutilate, I + lactofen, I + lenacil, I + linuron, I + mecoprop, I + mecoprop-P, I + mefenacet, I + mefluidide, I + mesosulfuron, I + mesosulfuron-methyl, I + mesotrione, I + metam, I + metamifop, I + metamitron, I + metazachlor, I + methabenzthiazuron, I + methazole, I + methylarsonic acid, I + methyldymron, I + methyl isothiocyanate, I + metolachlor, I + S-metolachlor, I + metosulam, I + metoxuron, I + metribuzin, I + metsulfuron, I + metsulfuron-methyl, I + molinate, I + monolinuron, I + naproanilide, I + napropamide, I + naptalam, I + neburon, I + nicosulfuron, I + n-methyl glyphosate, I + nonanoic acid, I + norflurazon, I + oleic acid (fatty acids), I + orbencarb, I + orthosulfamuron, I + oryzalin, I + oxadiargyl, I + oxadiazon, I + oxasulfuron, I + oxaziclomefone, I + oxyfluorfen, I + paraquat, I + paraquat dichloride, I + pebulate, I + pendimethalin, I + penoxsulam, I + pentachlorophenol, I + pentanochlor, I + pentoxazone, I + pethoxamid, I + phenmedipham, I + picloram, I + picolinafen, I + pinoxaden, I + piperophos, I + pretilachlor, I + primisulfuron, I + primisulfuron-methyl, I + prodiamine, I + profoxydim, I + prohexadione-calcium, I + prometon, I + prometryn, I + propachlor, I + propanil, I + propaquizafop, I + propazine, I + propham, I + propisochlor, I + propoxycarbazone, I + propoxycarbazone-sodium, I + propyzamide, I + prosulfocarb, I + prosulfuron, I + pyraclonil, I + pyraflufen, I + pyraflufen-ethyl, I + pyrasulfotole, I + pyrazolynate, I + pyrazosulfuron, I + pyrazosulfuron-ethyl, I + pyrazoxyfen, I + pyribenzoxim, I + pyributicarb, I + pyridafol, I + pyridate, I + pyriftalid, I + pyriminobac, I + pyriminobac-methyl, I + pyrimisulfan, I + pyrithiobac, I + pyrithiobac-sodium, I + pyroxasulfone, I + pyroxsulam, I + quinclorac, I + quinmerac, I + quinoclamine, I + quizalofop, I + quizalofop-P, I + rimsulfuron, I + saflufenacil, I + sethoxydim, I + siduron, I + simazine, I + simetryn, I + sodium chlorate, I + sulcotrione, I + sulfentrazone, I + sulfometuron, I + sulfometuron-methyl, I + sulfosate, I + sulfosulfuron, I + sulfuric acid, I + tebuthiuron, I + tefuryltrione, I + tembotrione, I + tepraloxydim, I + terbacil, I + terbumeton, I + terbuthylazine, I + terbutryn, I + thenylchlor, I + thiazopyr, I + thifensulfuron, I + thiencarbazone, I + thifensulfuron-methyl, I + thiobencarb, I + topramezone, I + tralkoxydim, I + tri-allate, I + triasulfuron, I + triaziflam, I + tribenuron, I + tribenuron-methyl, I + triclopyr, I + trietazine, I + trifloxysulfuron, I + trifloxysulfuron-sodium, I + trifluralin, I + triflusulfuron, I + triflusulfuron-methyl, I + trihydroxytriazine, I + trinexapac-ethyl, I + tritosulfuron, I + [3-[2-chloro-4-fluoro-5-(1-methyl-6-trifluoromethyl-2,4-dioxo-1,2,3,4-tetrahydropyrimidin-3-yl)phenoxy]-2-pyridyloxy]acetic acid ethyl ester (CAS RN 353292-31-6). The compounds of formula (I) and/or compositions of the present invention may also be combined with herbicidal compounds disclosed in WO06/024820 and/or WO07/096576.

The mixing partners of the compound of Formula (I) may also be in the form of esters or salts, as mentioned e.g. in The Pesticide Manual, Sixteenth Edition, British Crop Protection Council, 2012.

The compound of Formula (I) can also be used in mixtures with other agrochemicals such as fungicides, nematicides or insecticides, examples of which are given in The Pesticide Manual *(supra).*

The mixing ratio of the compound of Formula (I) to the mixing partner is preferably from 1: 100 to 1000:1.

The mixtures can advantageously be used in the above-mentioned formulations (in which case "active ingredient" relates to the respective mixture of compound of Formula I with the mixing partner).

The compounds of Formula (I) as described herein can also be used in combination with one or more safeners. Likewise, mixtures of a compound of Formula (I) as described herein with one or more further herbicides can also be used in combination with one or more safeners. The safeners can be AD 67 (MON 4660), benoxacor, cloquintocet-mexyl, cyprosulfamide (CAS RN 221667-31-8), dichlormid, fenchlorazole-ethyl, fenclorim, fluxofenim, furilazole and the corresponding R isomer, isoxadifen-ethyl, mefenpyr-diethyl, oxabetrinil, N-isopropyl-4-(2-methoxy-benzoylsulfamoyl)-benzamide (CAS RN 221668-34-4). Other possibilities include safener compounds disclosed in, for example, EP0365484 e.g N-(2-methoxybenzoyl)-4-[(methylaminocarbonyl)amino]benzenesulfonamide. Particularly preferred are mixtures of a compound of Formula I with cyprosulfamide, isoxadifen-ethyl, cloquintocet-mexyl and/or N-(2-methoxybenzoyl)-4-[(methyl-aminocarbonyl)amino]benzenesulfonamide.

The safeners of the compound of Formula (I) may also be in the form of esters or salts, as mentioned e.g. in The Pesticide Manual *(supra).* The reference to cloquintocet-mexyl also applies to a lithium, sodium, potassium, calcium, magnesium, aluminium, iron, ammonium, quaternary ammonium, sulfonium or phosphonium salt thereof as disclosed in WO 02/34048, and the reference to fenchlorazole-ethyl also applies to fenchlorazole, etc.

Preferably the mixing ratio of compound of Formula (I) to safener is from 100:1 to 1:10, especially from 20:1 to 1:1.

The mixtures can advantageously be used in the above-mentioned formulations (in which case "active ingredient" relates to the respective mixture of compound of Formula (I) with the safener).

As described above, compounds of formula (I) and/or compositions comprising such compounds may be used in methods of controlling unwanted plant growth, and in particular in controlling unwanted plant growth in crops of useful plants. Thus, the present invention further provides a method of selectively controlling weeds at a locus comprising crop plants and weeds, wherein the method comprises application to the locus, of a weedcontrolling amount of a compound of formula (I), or a composition as described herein. 'Controlling' means killing, reducing or retarding growth or preventing or reducing germination. Generally the plants to be controlled are unwanted plants (weeds). 'Locus' means the area in which the plants are growing or will grow.

The rates of application of compounds of Formula (I) may vary within wide limits and depend on the nature of the soil, the method of application (pre- or post-emergence; seed dressing; application to the seed furrow; no tillage application etc.), the crop plant, the weed(s) to be controlled, the prevailing climatic conditions, and other factors governed by the method of application, the time of application and the target crop. The compounds of Formula I according to the invention are generally applied at a rate of from 10 to 2000 g/ha, especially from 50 to 1000 g/ha.

The application is generally made by spraying the composition, typically by tractor mounted sprayer for large areas, but other methods such as dusting (for powders), drip or drench can also be used.

Useful plants in which the composition according to the invention can be used include crops such as cereals, for example barley and wheat, cotton, oilseed rape, sunflower, maize, rice, soybeans, sugar beet, sugar cane and turf.

Crop plants can also include trees, such as fruit trees, palm trees, coconut trees or other nuts. Also included are vines such as grapes, fruit bushes, fruit plants and vegetables.

Crops are to be understood as also including those crops which have been rendered tolerant to herbicides or classes of herbicides (e.g. ALS-, GS-, EPSPS-, PPO-, ACCase- and HPPD-inhibitors) by conventional methods of breeding or by genetic engineering. An example of a crop that has been rendered tolerant to imidazolinones, e.g. imazamox, by conventional methods of breeding is Clearfield® summer rape (canola). Examples of crops that have been rendered tolerant to herbicides by genetic engineering methods include e.g. glyphosate- and glufosinate-resistant maize varieties commercially available under the trade names RoundupReady® and LibertyLink®, as well as those where the crop plant has been engineered to over-express homogentisate solanesyltransferase as taught in, for example, WO2010/029311.

Crops are also to be understood as being those which have been rendered resistant to harmful insects by genetic engineering methods, for example Bt maize (resistant to European corn borer), Bt cotton (resistant to cotton boll weevil) and also Bt potatoes (resistant to Colorado beetle). Examples of Bt maize are the Bt 176 maize hybrids of NK® (Syngenta Seeds). The Bt toxin is a protein that is formed naturally by *Bacillus thuringiensis* soil bacteria. Examples of toxins, or transgenic plants able to synthesise such toxins, are described in EP-A-451 878, EP-A-374 753, WO 93/07278, WO 95/34656, WO 03/052073 and EP-A-427 529. Examples of transgenic plants comprising one or more genes that code for an insecticidal resistance and express one or more toxins are KnockOut® (maize), Yield Gard® (maize), NuCOTIN33B® (cotton), Bollgard® (cotton), NewLeaf® (potatoes), NatureGard® and Protexcta®. Plant crops or seed material thereof can be both resistant to herbicides and, at the same time, resistant to insect feeding ("stacked" transgenic events). For example, seed can have the ability to express an insecticidal Cry3 protein while at the same time being tolerant to glyphosate.

Crops are also to be understood to include those which are obtained by conventional methods of breeding or genetic engineering and contain so-called output traits (e.g. improved storage stability, higher nutritional value and improved flavour).

Other useful plants include turf grass for example in golf-courses, lawns, parks and roadsides, or grown commercially for sod, and ornamental plants such as flowers or bushes.

The compositions can be used to control unwanted plants (collectively, 'weeds'). The weeds to be controlled include both monocotyledonous (e.g. grassy) species, for example: *Agrostis, Alopecurus, Avena, Brachiaria, Bromus, Cenchrus, Cyperus, Digitaria, Echinochloa, Eleusine, Lolium, Monochoria, Rottboellia, Sagittaria, Scirpus, Setaria and Sorghum;* and dicotyledonous species, for example: *Abutilon, Amaranthus, Ambrosia, Chenopodium, Chrysanthemum, Conyza, Galium, Ipomoea, Kochia, Nasturtium,* *Polygonum, Sida, Sinapis, Solanum, Stellaria, Veronica, Viola* and *Xanthium.* Weeds can also include plants which may be considered crop plants but which are growing outside a crop area ('escapes'), or which grow from seed left over from a previous planting of a different crop ('volunteers'). Such volunteers or escapes may be tolerant to certain other herbicides.

Preferably the weeds to be controlled and/or growth-inhibited, include monocotyledonous weeds, more preferably grassy monocotyledonous weeds, in particular those from the following genus: *Agrostis, Alopecurus, Apera, Avena, Brachiaria, Bromus, Cenchrus, Cyperus* (a genus of sedges), *Digitaria, Echinochloa, Eleusine, Eriochloa, Fimbristylis* (a genus of sedges), *Juncus* (a genus of rushes), *Leptochloa, Lolium, Monochoria, Ottochloa, Panicum, Pennisetum, Phalaris, Poa, Rottboellia, Sagittaria, Scirpus* (a genus of sedges), *Setaria* and/or *Sorghum,* and/or volunteer corn (volunteer maize) weeds; in particular: *Alopecurus myosuroides* (ALOMY, English name "blackgrass"), *Apera spica-venti, Avena fatua* (AVEFA, English name "wild oats"), *Avena ludoviciana, Avena sterilis, Avena sativa* (English name "oats" (volunteer)), *Brachiaria decumbens, Brachiaria plantaginea, Brachiaria platyphylla* (BRAPP), *Bromus tectorum, Digitaria horizontalis, Digitaria insularis, Digitaria sanguinalis* (DIGSA), *Echinochloa crusgalli* (English name "common barnyard grass", ECHCG), *Echinochloa oryzoides, Echinochloa colona or colonum, Eleusine indica, Eriochloa villosa* (English name "woolly cupgrass"), *Leptochloa chinensis, Leptochloa panicoides, Lolium perenne* (LOLPE, English name "perennial ryegrass"), *Lolium multiflorum* (LOLMU, English name "Italian ryegrass"), *Lolium persicum* (English name "Persian darnel"), *Lolium rigidum, Panicum dichotomiflorum* (PANDI), *Panicum miliaceum* (English name "wild proso millet"), *Phalaris minor, Phalaris paradoxa, Poa annua* (POAAN, English name "annual bluegrass"), *Scirpus maritimus, Scirpus juncoides*, *Setaria viridis* (SETVI, English name "green foxtail"), *Setaria faberi* (SETFA, English name "giant foxtail"), *Setaria glauca*, *Setaria lutescens* (English name "yellow foxtail"), *Sorghum bicolor,* and/or *Sorghum halepense* (English name "Johnson grass"), and/or *Sorghum vulgare;* and/or volunteer corn (volunteer maize) weeds.

In one embodiment, grassy monocotyledonous weeds to be controlled comprise weeds from the genus: *Agrostis, Alopecurus, Apera, Avena, Brachiaria, Bromus, Cenchrus, Digitaria, Echinochloa, Eleusine, Eriochloa, Leptochloa, Lolium, Ottochloa, Panicum, Pennisetum, Phalaris, Poa, Rottboellia, Setaria* and/or *Sorghum,* and/or volunteer corn (volunteer maize) weeds; in particular: weeds from the genus *Agrostis, Alopecurus, Apera, Avena, Brachiaria, Bromus, Cenchrus, Digitaria, Echinochloa, Eleusine, Eriochloa, Leptochloa, Lolium, Panicum, Phalaris, Poa, Rottboellia, Setaria,* and/or *Sorghum,* and/or volunteer corn (volunteer maize) weeds.

In a further embodiment, the grassy monocotyledonous weeds are "warm-season" (warm climate) grassy weeds; in which case they preferably comprise (e.g. are): weeds from the genus *Brachiaria, Cenchrus, Digitaria, Echinochloa, Eleusine, Eriochloa, Leptochloa, Ottochloa, Panicum, Pennisetum, Phalaris, Rottboellia, Setaria* and/or *Sorghum,* and/or volunteer corn (volunteer maize) weeds. More preferably, the grassy monocotyledonous weeds, e.g. to be controlled and/or growth-inhibited, are "warm-season" (warm climate) grassy weeds comprising (e.g. being): weeds from the genus *Brachiaria, Cenchrus, Digitaria, Echinochloa, Eleusine, Eriochloa, Panicum, Setaria* and/or *Sorghum,* and/or volunteer corn (volunteer maize) weeds.

In another particular embodiment the grassy monocotyledonous weeds, are "coolseason" (cool climate) grassy weeds; in which case they typically comprise weeds from the genus *Agrostis, Alopecurus, Apera, Avena, Bromus, Lolium* and/or Poa.

Various aspects and embodiments of the present invention will now be illustrated in more detail by way of example. Novel methods and intermediates described therein are considered further aspects of the invention. It will be appreciated that modification of detail may be made without departing from the scope of the invention.

### PREPARATION EXAMPLES

Those skilled in the art will appreciate that depending on the nature of the substituents X¹, X², X³, R¹, R², R³, R⁴, R⁵, R⁶, R⁷, R⁸, R⁹, R¹⁰, R¹¹, R¹², R¹³, R¹⁴, R^{a}, R^{b}, n, p, q r and s, compounds of Formula (I) may exist in different interconvertible rotameric forms as described in, for example S.A. Richards and J.C. Hollerton, Essential Practical NMR for Organic Chemistry, John Wiley and sons (2010). For clarity, only the spectroscopic data for the major rotameric form is quoted.

### General Methods

[Pd(IPr*)(cin)Cl] refers to chlorophenylallyl[1,3-bis[2,6-bis(diphenylmethyl)-4-methylphenyl-imidazol-2-ylidene]palladium(II) [1380314-24-8] ― see Chem. Eur. J. 2012, 18, 4517
JackiePhos Pd G3 refers to methanesulfonato[2-bis(3,5-di(trifluoromethyl)-phenylphosphino)-3,6-dimethoxy-2',4',6'-tri-i-propyl-1,1'-biphenyl](2'-amino-1,1'-biphenyl-2-yl)palladium(II) ― see J. Am. Chem. Soc., 2009, 131, 16720.
tBuBrettPhos Pd G3 refers to methanesulfonato(2-(di-t-butylphosphino)-3,6-dimethoxy-2',4',6'-tri-i-propyl-1,1'-biphenyl)(2'-amino-1,1'-biphenyl-2-yl)palladium(II), dichloromethane adduct [1536473-72-9] ― see Org. Lett., 2013, 15, 1394

### EXAMPLE P1: Synthesis of tert-butyl N-[2-(5-fluoro-3-pyridyl)-4-methyl-pyrimidin-5-yl]carbamate (compound E1)

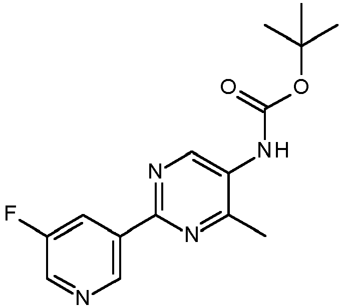

### Step 1: Synthesis of tert-butyl N-(2-chloro-4-methyl-pyrimidin-5-yl)carbamate

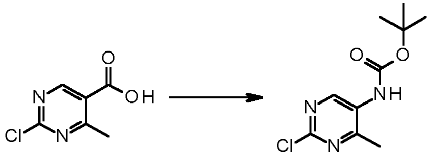

To a stirred solution of 2-chloro-4-methyl-pyrimidine-5-carboxylic acid (500 mg, 2.90 mmol) and triethylamine (0.525 mL, 3.77 mmol) in tert-butanol (25 mL) was added diphenylphosphoryl azide (0.81 mL, 3.77 mmol). The reaction mixture heated to 90°C for 4 hours and then allowed to cool to RT overnight. The reaction mixture was diluted with water (100 mL) and extracted with EtOAc (3 x 25 mL). The combined organic extracts were washed with brine (20 mL), dried over MgSO₄ and evaporated to dryness under reduced pressure to give a yellow oil. The crude product was purified by flash chromatography on silica gel using an EtOAc/isohexane gradient as eluent to give the desired product (527mg, 75%) as a white solid.
¹H NMR (400MHz, CDCl₃) δ = 9.03 (br s, 1H), 6.42 (br s, 1H), 2.50 (s, 3H), 1.53 (s, 9H).

### Step 2: Synthesis of tert-butyl N-[2-(5-fluoro-3-pyridyl)-4-methyl-pyrimidin-5-yl]carbamate (compound E1)

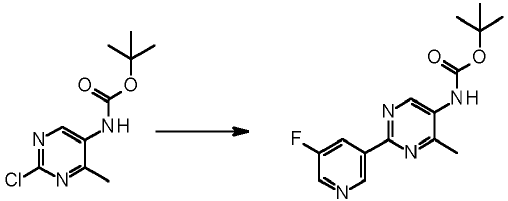

A mixture of tert-butyl N-(2-chloro-4-methyl-pyrimidin-5-yl)carbamate (212 mg, 0.87 mmol), 5-fluoropyridine-3-boronic acid (160mg, 1.09 mmol), potassium carbonate (265mg, 1.91 mmol) and [Pd(IPr*)(cin)Cl) (50mg, 0.043 mmol) in EtOH (6.40 mL) was heated at 80°C under an N₂ atmosphere for 1 hour. The mixture was filtered through celite, washed through with EtOH and evaporated to dryness under reduced pressure to give an orange-brown gum. The crude product was purified by flash chromatography on silica gel using an EtOAc/isohexane gradient as eluent to give the desired product (170mg, 64%) as a yellow solid.
¹H NMR (400MHz, CDCl₃) δ = 9.42 (s, 1H), 9.20 (br s, 1H), 8.52 (d, 1H), 8.38 (dd, 1H), 6.55 (br s, 1H), 2.57 (s, 3H), 1.55 (s, 9H).

### EXAMPLE P2: Synthesis of tert-butyl N-[2-(5-fluoro-3-pyridyl)-4-methyl-pyrimidin-5-yl]-N-methyl-carbamate (compound E2)

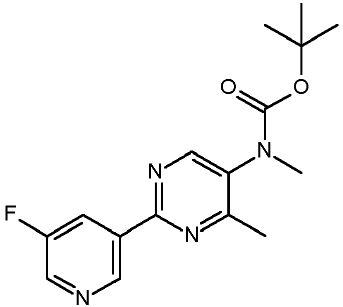

### Step 1: Synthesis of tert-butyl N-[2-(5-fluoro-3-pyridyl)-4-methyl-pyrimidin-5-yl]-N-methyl-carbamate (compound E2)

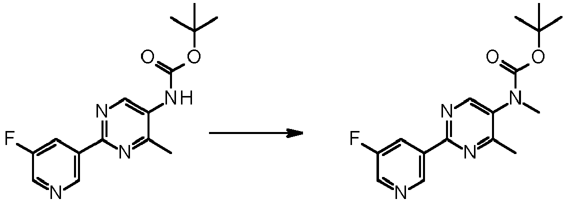

To a stirred solution of tert-butyl N-[2-(5-fluoro-3-pyridyl)-4-methyl-pyrimidin-5-yl]carbamate (246 mg, 0.81 mmol) in tetrahydrofuran (5 mL) at RT under an N₂ atmosphere was added in a single portion NaH (60% dispersion in mineral oil)(34mg, 0.85 mmol). The reaction was then stirred at RT for 20 minutes and then iodomethane (0.051 mL, 0.81 mmol) was added and the reaction was stirred for 1 hour. The reaction was quenched with H₂O (5 mL) and HCl (to acidic pH) was added and the reaction extracted with EtOAc (3 x 10 mL). The combined organic extracts were washed with brine (10 mL), dried over MgSO₄ and evaporated to dryness under reduced pressure. The crude product was purified by flash chromatography on silica gel using an EtOAc/isohexane gradient as eluent to give the desired product (257mg, 81%) as a yellow solid.
¹H NMR (400MHz, CDCl₃) δ = 9.47 (br s, 1H), 8.63 - 8.48 (m, 2H), 8.42 (br d, 1H), 3.23 (s, 3H), 2.53 (s, 3H), 1.60 - 1.28 (br m, 9H).

### EXAMPLE P3: Synthesis of tert-butyl N-(4-methyl-2-pyrimidin-5-yl-pyrimidin-5-yl)carbamate (compound E7)

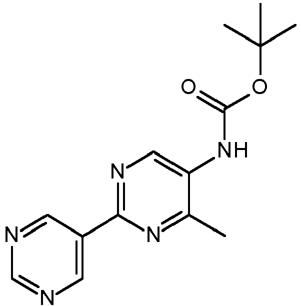

### Step 1: Synthesis of tert-butyl N-(4-methyl-2-pyrimidin-5-yl-pyrimidin-5-yl)carbamate

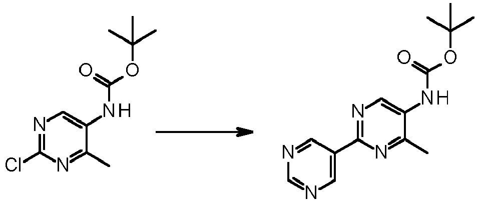

A mixture of tert-butyl N-(2-chloro-4-methyl-pyrimidin-5-yl)carbamate (1.93 g, 7.92 mmol), pyrimidin-5-ylboronic acid (1.47g, 11.9 mmol), K₂CO₃ (2.41g, 17.4 mmol) and [Pd(IPr*)(cin)Cl) (0.464g, 0.40 mmol) in EtOH (40 mL) was heated at 80°C for 1.5 hours. The reaction was cooled to RT, filtered through celite, washed through with EtOH and evaporated to dryness under reduced pressure to give an orange-brown gum. The crude product was purified by flash chromatography on silica gel using an EtOAc/isohexane gradient as eluent to give the desired product (1.69g, 74%) as a pale yellow solid.
¹H NMR (400MHz, CDCl₃) δ = 9.65 (s, 2H), 9.27 (s, 1H), 9.25 (br s, 1H), 6.38 (br s, 1H), 2.58 (s, 3H), 1.56 (s, 9H).

### Example P4: Synthesis of tert-butyl N-[2-(5-fluoro-3-pyridyl)-4-(trifluoromethyl)pyrimidin-5-yl]carbamate (compound E4)

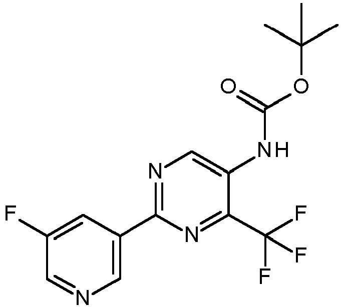

### Route 1, Step 1: Synthesis of tert-butyl N-[2-chloro-4-(trifluoromethyl)pyrimidin-5-yl]carbamate

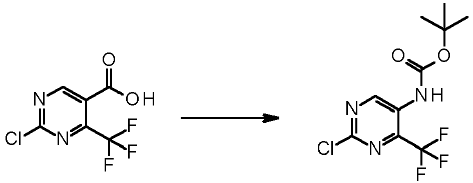

To a stirred solution of 2-chloro-4-(trifluoromethyl)pyrimidine-5-carboxylic acid (0.95g, 4.19 mmol) and triethylamine (0.76 mL, 5.45 mmol) in tert-butanol (7.5 mL) and toluene (5 mL) at reflux was added dropwise a solution of diphenylphosphoryl azide (1.17 mL, 5.45 mmol) in toluene (2.5 mL). The reaction was heated at reflux for 2 hours and then allowed to cool to RT. The reaction mixture was diluted with EtOAc (100 mL), washed with brine (100 mL). The aqueous phase was extracted with further EtOAc (2 x 100 mL), the combined organic extracts were dried over MgSO₄ and evaporated to dryness under reduced pressure. The crude product was purified by flash chromatography on silica gel using an EtOAC/isohexane gradient as eluent to give the desired product (0.46g, 37%) as a colourless solid.
¹H NMR (400 MHz, CDCl₃) δ 9.68 (s, 1H), 6.80 (br s, 1H), 1.55 (s, 9H).

### Route 1, Step 2: Synthesis of tert-butyl N-[2-(5-fluoro-3-pyridyl)-4-(trifluoromethyl)pyrimidin-5-yl]carbamate

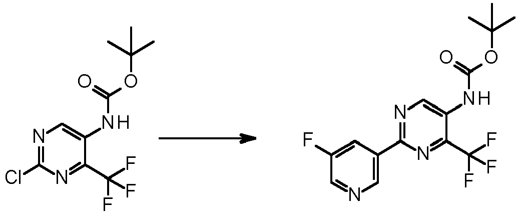

A mixture of tert-butyl N-[2-chloro-4-(trifluoromethyl)pyrimidin-5-yl]carbamate (0.20g, 0.672 mmol) and (5-fluoro-3-pyridyl)boronic acid (0.133g, 0.941 mmol) in EtOH (0.54 mL), toluene (2 mL) and water (0.92 mL) was sparged with N₂ for 30 min at RT. K₂CO₃ (0.186g, 1.34 mmol) and Pd(PPh₃)₄ (0.039g, 0.0336 mmol) were then added and the yellow solution heated to 85°C under an N₂ atmosphere for 8 hours and cooled to RT overnight. The mixture was diluted with EtOAc (30 mL) and washed with brine (30mL). The aqueous layer was then extracted with further EtOAc (2 x 30 mL) and the combined organic extracts were dried over MgSO₄ and evaporated to dryness under reduced pressure. The crude product was purified by flash chromatography on silica gel using an EtOAc/isohexane gradient as eluent to give the desired product (0.224g, 93%) as a colourless solid.
¹H NMR (400 MHz, CDCl₃) δ 9.81 (s, 1H), 9.45 (s, 1H), 8.58 (d, 1H), 8.39 (d, 1H), 6.97 (br. s, 1H), 1.58 (m, 9H)

### Route 2, Step 1: Synthesis of ethyl (2E/Z)-2-(ethoxymethylene)-4,4,4-trifluoro-3-oxo-butanoate

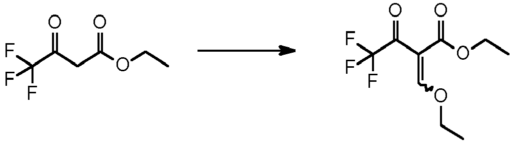

A mixture of acetic anhydride (30.8 mL, 325.9mmol), triethyl orthoformate (36.1 mL, 217.3 mmol) and ethyl 4,4,4-trifluoro-3-oxo-butanoate (15.89 mL, 108.6mmol) was heated at reflux for 6 hours and then allowed to cool to RT. The excess reagents were removed by distillation under reduced pressure to leave the desired product as a mixture of E/Z isomers (23.2g, 89%) as an orange oil.
¹H NMR (400MHz, CDCl₃) δ 7.83 and 7.72 (2 x s, 1H), 4.40-4.20 (m, 4H), 1.49-1.22 (m, 3H).

### Route 2, Step 2: Synthesis of (5-fluoropyridine-3-carboximidoyl)ammonium chloride

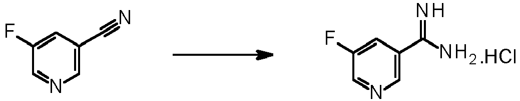

To a stirred solution of 5-fluoropyridine-3-carbonitrile (2.0 g, 16.38 mmol) in methanol (20 mL) at RT was added NaOMe (88mg, 1.64 mmol) and the reaction stirred at RT overnight. Ammonium chloride (1.40g, 26.21 mmol) was added in a single portion and the reaction mixture stirred overnight at RT. The reaction mixture was filtered and the filtrate concentrated to dryness under reduced pressure. The residue was suspended in EtOH (50 mL) and then heated at reflux. The undissolved solid was filtered off and the filtrate concentrated to 1/3 of its volume and then left to stand at RT. The resultant crystals were filtered off, washed with EtOH and air-dried to give the desired product (2.11 g, 73%) as white crystals.
¹H NMR (400 MHz, d6-DMSO) δ 8.93 (d, 1H), 8.88 (s, 1H), 8.29-8.23 (m, 1H).

### Route 2, Step 3: Synthesis of ethyl 2-(5-fluoro-3-pyridyl)-4-(trifluoromethyl)pyrimidine-5-carboxylate

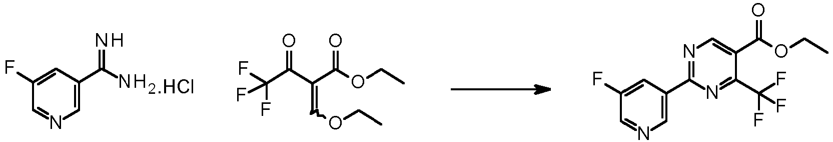

To a stirred solution of (5-fluoropyridine-3-carboximidoyl)ammonium chloride (2.0g, 11.4 mmol) and ethyl (2E/Z)-2-(ethoxymethylene)-4,4,4-trifluoro-3-oxo-butanoate (2.74g, 11.4 mmol) in EtOH (40 mL) was added NaOEt (1.16g, 17.1 mmol). The reaction was heated at reflux for 2hrs and then further NaOEt (155mg, 0.2 equiv) was added, the reaction mixture heated at reflux for a further 1hr and then allowed to cool to RT overnight. The reaction mixture was evaporated to dryness under reduced pressure and the residue partitioned between water (25 mL) and extracted with EtOAc (3 x 20 mL). The combined organic extracts were washed with brine (20 mL), dried over MgSO₄ and evaporated to dryness under reduced pressure to give an orange oil. The crude product was purified by flash chromatography on silica gel using a gradient of 5-25% ethyl acetate in isohexane as eluent to give the desired product (2.15g, 60%) as an off-white solid.
¹H NMR (400 MHz, CDCl₃) δ 9.57 (s, 1H), 9.32 (s, 1H), 8.66 (d, 1H), 8.52-8.47 (m, 1H), 4.49 (q, 2H), 1.44 (t, 3H).

### Route 2, Step 4: Synthesis of 2-(5-fluoro-3-pyridyl)-4-(trifluoromethyl)pyrimidine-5-carboxylic acid

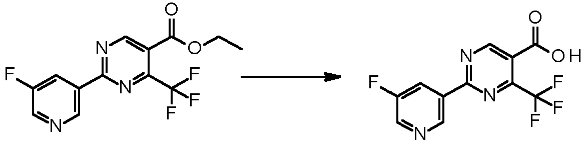

To a stirred solution of ethyl 2-(5-fluoro-3-pyridyl)-4-(trifluoromethyl)pyrimidine-5-carboxylate (2.15g, 6.82 mmol) in EtOH (60 mL) and water (20 mL) was added LiOH (0.49g, 20.5 mmol). The reaction was stirred at RT for 3 hours. The EtOH was removed under reduced pressure and the resulting residue diluted with water (30 mL), acidified with 2M HCl to pH 5 and extracted with EtOAc (3 x 25 mL). The combined organic extracts were washed with brine (15 mL), dried over MgSO₄ and evaporated to dryness under reduced pressure to give the desired product (1.34 g, 68%) as a white powder.
¹H NMR (400 MHz, d6-DMSO) δ 9.50 (s, 1H), 9.41 (s, 1H), 8.88 (s, 1H), 8.52-8.45 (m, 1H).

### Route 2, Step 5: Synthesis of tert-butyl N-[2-(5-fluoro-3-pyridyl)-4-(trifluoromethyl)pyrimidin-5-yl]carbamate (compound E4)

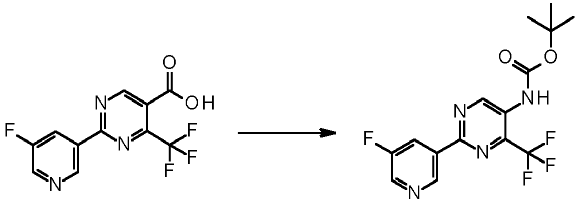

To a stirred solution of 2-(5-fluoro-3-pyridyl)-4-(trifluoromethyl)pyrimidine-5-carboxylic acid (1.30g, 4.53 mmol) and triethylamine (0.82 mL, 5.89 mmol) in tert-butanol (12 mL) and toluene (6mL) at reflux was added dropwise over 5 minutes a solution of diphenylphosphoryl azide (1.27 mL, 5.89 mmol) in toluene (6 mL). The reaction was heated at reflux for 3 hours, then allowed to cool to RT, diluted with EtOAc (60mL) and washed with brine (60mL). The aqueous layer was extracted with further EtOAc (2 x 100mL). The combined organic extracts were dried over MgSO₄ and evaporated to dryness under reduced pressure. The crude product was purified by flash chromatography on silica gel using an EtOAc/isohexane gradient as eluent to give the desired product (941 mg, 59%) as a colourless solid
¹H NMR (400 MHz, CDCl₃) δ 9.81 (s, 1H), 9.45 (s, 1H), 8.58 (d, 1H), 8.39 (d, 1H), 6.97 (br s, 1H), 1.58 (m, 9H)

### EXAMPLE P5: Synthesis of N-[2-(5-fluoro-3-pyridyl)-4-(trifluoromethyl)-pyrimidin-5-yl]morpholine-4-carboxamide (compound E8)

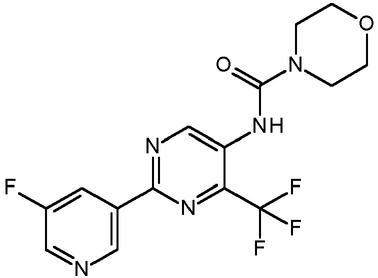

### Step 1: Synthesis of 2-(5-fluoro-3-pyridyl)-4-(trifluoromethyl)pyrimidin-5-amine

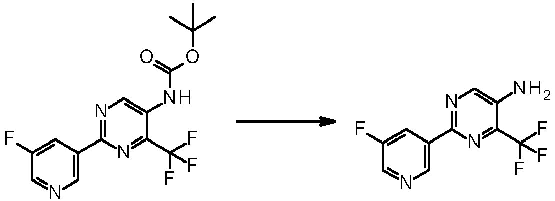

To a stirred solution of tert-butyl N-[2-(5-fluoro-3-pyridyl)-4-(trifluoromethyl)pyrimidin-5-yl]carbamate (0.94 g, 2.62 mmol) in DCM (10 mL) was added dropwise a solution of TFA (2.03 mL, 26.23 mmol) in DCM (10mL). The reaction mixture was heated at reflux for 5hrs and allowed to cool to RT. The reaction mixture was added slowly and portionwise to a stirred solution of saturated aqueous sodium bicarbonate solution (15 mL) and then extracted with DCM (5 x 20 mL). The combined organic extracts were dried and evaporated to dryness under reduced pressure to give the desired product (590 mg, 87%) as a pale yellow powder.
¹H NMR (400 MHz, CDCl₃) δ 9.37 (s, 1H), 8.53-8.46 (m, 2H), 8.30 (d,1H), 4.44 (br s, 2H).

### Step 2: Synthesis of N-[2-(5-fluoro-3-pyridyl)-4-(trifluoromethyl)pyrimidin-5-yl]morpholine-4-carboxamide (compound E8)

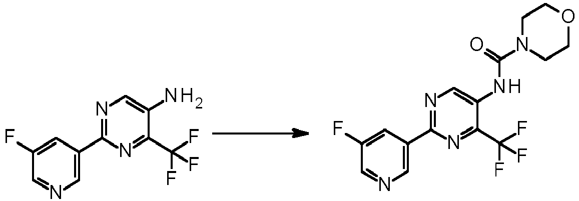

To a stirred solution of 2-(5-fluoro-3-pyridyl)-4-(trifluoromethyl)pyrimidin-5-amine (150mg, 0.581 mmol), pyridine (0.188 mL, 2.32 mmol) and DMAP (7mg, 0.06 mmol) in DCM (5 mL) was added a solution of (4-nitrophenyl) chloroformate (234mg, 1.16 mmol) in DCM (5 mL) in one portion. The reaction was stirred at RT for 2.5 hours then morpholine (0.254 mL, 2.91 mmol) was added and the reaction mixture was allowed to stir overnight. The reaction mixture was evaporated to dryness under reduced pressure and the residue purified by flash chromatography on silica gel using an EtOAc/isohexane gradient as eluent. The crude product was further purified by suspending in CHCl₃ and filtered through a plug of celite to give the desired product (98mg, 45%) as a colourless solid.
¹H NMR (400MHz, DMSO-d₆) δ 9.45 (s, 1H), 9.37 (s, 1H), 8.59 (d, 1H), 8.40 (d, 1H), 7.99 (s, 1H), 3.79-3.70 (m, 4H), 3.61-3.52 (m, 4H).

### EXAMPLE P6: Synthesis of 1-[2-(5-fluoro-3-pyridyl)-4-(trifluoromethyl)-pyrimidin-5-yl]-3-methyl-imidazolidin-2-one (compound E9).

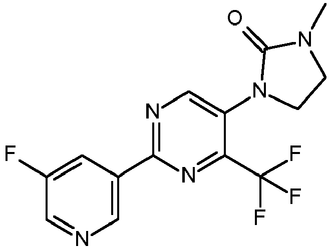

### Step 1: Synthesis of 5-chloro-2-methylsulfanyl-4-(trifluoromethyl)pyrimidine

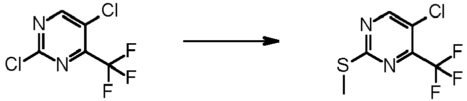

To a stirred suspension of NaSMe (0.17g, 2.42 mmol) in MeOH (5 mL) at RT was added 2,5-dichloro-4-(trifluoromethyl)pyrimidine (0.50g, 2.30 mmol). The reaction was heated at reflux for 2 ¼ hours, allowed to cool to RT and then evaporated to dryness under reduced pressure to give a pale yellow paste. The crude material was dissolved in EtOAc (20 mL) and washed with H₂O (3 × 20 mL). The organic phase was then dried over MgSO₄ and evaporated to dryness under reduced pressure to give a pale yellow oil. The crude product was purified by flash chromatography on silica gel using an EtOAc/isohexane gradient as eluent to give the desired product (0.419g, 80%) as a colourless oil.
¹H NMR (400 MHz, CDCl₃) δ 8.66 (s, 1H), 2.60 (s, 3H).

### Step 2: Synthesis of 5-chloro-2-(5-fluoro-3-pyridyl)-4-(trifluoromethyl)pyrimidine

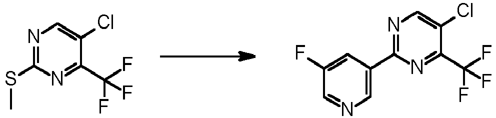

A microwave vial was charged with 5-chloro-2-methylsulfanyl-4-(trifluoromethyl)pyrimidine (750mg, 3.28 mmol), (5-fluoro-3-pyridyl)boronic acid (693mg, 4.92 mmol), tris(2-furyl)phosphane (122mg, 0.525 mmol), copper(l) 3-methylsalicylate (1.76g, 8.20 mmol), Pd₂dba₃ (120mg, 0.131 mmol) and anhydrous THF (20 mL) (pre-sparged with N₂ for 30 min) and heated at 100°C for 1 hour under microwave irradiation. The reaction mixture was evaporated to dryness under reduced pressure and the residue was taken up in Et₂O (200 mL) and washed with aq. NH₄OH (2:1 conc.:water, 3 x 200mL) and brine (200mL). The organic phase was dried over MgSO₄ and evaporated to dryness under reduced pressure. The crude product was purified twice by flash chromatography on silica gel, first with an EtOAc/isohexane gradient as eluent followed by a 0-10% MeOH/DCM gradient as eluent to give the desired product (603mg, 66%) as a crystalline pale yellow solid.
¹H NMR (400 MHz, CDCl₃): δ 9.49 (s, 1H), 9.01 (s, 1H), 8.63 (d, 1H), 8.47-8.39 (m, 1H).

### Step 3: Synthesis of 1-[2-(5-fluoro-3-pyridyl)-4-(trifluoromethyl)pyrimidin-5-yl]-3-methyl-imidazolidin-2-one (compound E9).

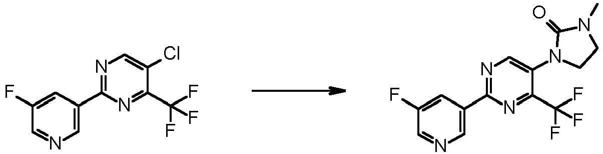

A microwave vial was charged with 5-chloro-2-(5-fluoro-3-pyridyl)-4-(trifluoromethyl)pyrimidine (120mg, 0.43 mmol), JackiePhos Pd G3 (20mg, 0.017 mmol), Cs₂CO₃ (282mg, 0.865 mmol), 1-methylimidazolidin-2-one (108mg, 1.08 mmol) and toluene (1 mL) and heated for 1 hour at 150°C under microwave irradiation. The reaction mixture was diluted with DCM (20 mL) and washed with water (20 mL). The aqueous layer was extracted with further portions of DCM (2 x 20 mL) and the combined organic extracts were evaporated to dryness under reduced pressure. The crude product was purified by flash chromatography on silica gel using an EtOAc/isohexane gradient as eluent to give the desired product (123mg, 83%) as a colourless solid.
¹H NMR (400 MHz, CDCl₃) δ 9.50 (s, 1H), 9.01 (s, 1H), 8.61 (d, 1H), 8.43 (d, 1H), 3.87-3.80 (m, 2H), 3.64-3.58 (m, 2H), 2.95 (s, 3H).

### EXAMPLE P7: Synthesis of tert-butyl N-[2-(5-cyano-3-pyridyl)-4-(trifluoromethyl)pyrimidin-5-yl]carbamate (compound E12)

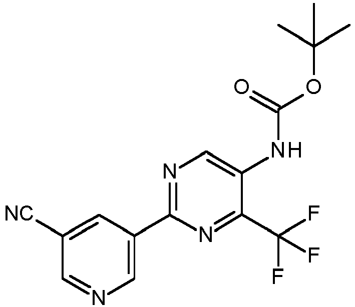

### Step 1: Synthesis of 5-[5-chloro-4-(trifluoromethyl)pyrimidin-2-yl]pyridine-3-carbonitrile

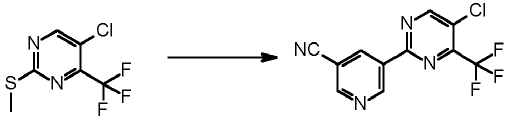

A microwave vial was charged with 5-chloro-2-methylsulfanyl-4-(trifluoromethy)pyrimidine (0.175g, 0.77 mmol), (5-cyano-3-pyridyl)boronic acid (0.170g, 1.15 mmol), Pd₂dba₃ (0.028g, 0.031 mmol), tris(2-furyl)phosphane (0.028g, 0.122 mmol), copper(l) 3-methylsalicylate (0.411g, 1.91 mmol) and THF (4.67 mL), capped and then degassed by evacuating and purging with N₂ three times. The reaction was heated at 100°C for 1 hour under microwave irradiation. The reaction mixture was diluted with Et₂O (25 mL) and washed with 1:2 water:conc. ammonia solution (10 mL). The aqueous phase was extracted with further Et₂O (2 × 25 mL) and the combined organic extracts were washed with 1:2 water:conc. ammonia solution (10 mL), brine (10 mL), dried over MgSO₄ and evaporated to dryness under reduced pressure to give a brown gum. The crude product was purified by flash chromatography on silica gel using an EtOAc/isohexane gradient as eluent to give the desired product (0.096g, 44%) as an off-white solid.
¹H NMR (400 MHz, CDCl₃): δ 9.84 (s, 1H), 9.08-8.98 (m, 3H).

### Step 2: Synthesis of tert-butyl N-[2-(5-cyano-3-pyridyl)-4-(trifluoromethyl)pyrimidin-5-yl]carbamate (compound E12)

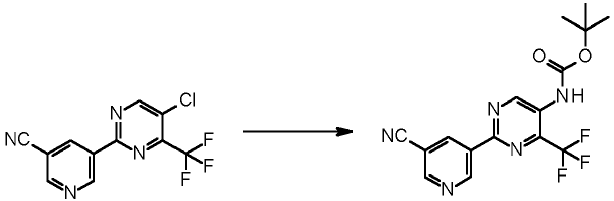

A microwave vial was charged with 5-[5-chloro-4-(trifluoromethyl)pyrimidin-2-yl]pyridine-3-carbonitrile (80mg, 0.25 mmol), sodium cyanate (37mg, 0.56 mmol), ^{t}BuBrettPhos Pd G3 (10mg, 0.011 mmol) and anhydrous t-BuOH (1.6 mL), capped and then degassed by evacuating and purging with N₂ three times. The reactions was heated at 140°C for 1 hour under microwave irradiation. The reaction mixture was evaporated to dryness under reduced pressure and the residue purified by flash chromatography on silica gel using an EtOAc/isohexane gradient as eluent to give the desired product (40mg, 39%) as an off white solid.
¹H NMR (400 MHz, CDCl₃): δ 9.89 (s, 1H), 9.81 (d, 1H), 8.99-8.94 (m, 2H), 6.92 (br s, 1H), 1.58 (s, 9H)

### EXAMPLE P8: Synthesis of isopropyl N-[2-(5-fluoro-3-pyridyl)-4-(trifluoromethyl)pyrimidin-5-yl]carbamate (compound E14)

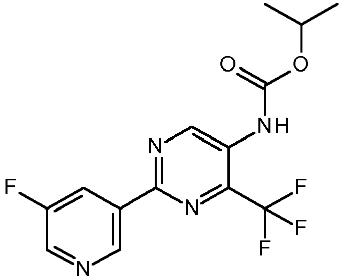

### Step 1: Synthesis of isopropyl N-[2-(5-fluoro-3-pyridyl)-4-(trifluoromethyl)pyrimidin-5-yl]carbamate (compound E14)

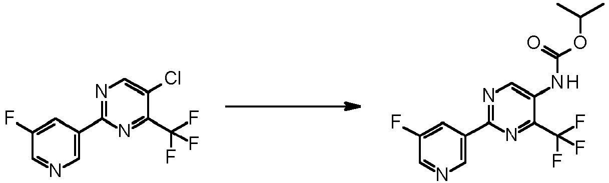

A microwave vial was charged with 5-chloro-2-(5-fluoro-3-pyridyl)-4-(trifluoromethyl)pyrimidine (200mg, 0.72 mmol), isopropyl carbamate (186mg, 1.80 mmol), Jackiephos Pd G3 (33mg, 0.029 mmol), Cs₂CO₃ (470mg, 1.44 mmol) and toluene (1 mL) and heated at 150°C for 1 hour under microwave irradiation. The reaction mixture was evaporated to dryness under reduced pressure and purified by flash chromatography on silica gel using an EtOAc/isohexane gradient as eluent to give the desired product (59mg, 24%) as a beige solid.
¹H NMR (400 MHz, CDCl₃): δ 9.84 (s, 1H), 9.46 (s, 1H), 8.59 (d, 1H), 8.42-8.37 (m, 1H), 6.95 (br s, 1H), 5.15-5.03 (m, 1H), 1.36 (d, 6H).

Further examples of the invention were made in an analogous manner using the methods described above in Examples P1 to P8, with respect to compounds E5, E6, E7, E10, E11, E13, E15, E16, E17, E18, E19, E20, E21 and E22. Table 2 below, shows the structure of these compounds and the physical characterising data obtained using one or more of methods A to C as outlined below.

**Table 2: Characterising data for Compounds of formula (I) made by the methods described above**

| **Compound** | **Structure** | **Data (400MHz, CDCl₃**) **unless stated** |
|---|---|---|
| E1 | 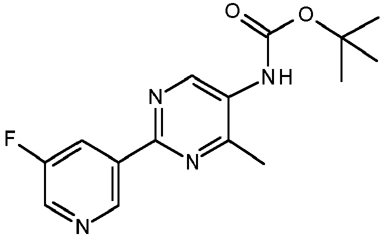 | 9.42 (s, 1H), 9.20 (br s, 1H), 8.52 (d, 1H), 8.38 (dd, 1H), 6.55 (br s, 1H), 2.57 (s, 3H), 1.55 (s, 9H) |
| E2 | 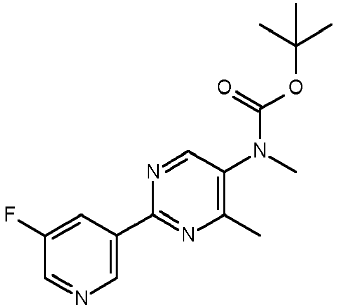 | 9.47 (br s, 1H), 8.63 - 8.48 (m, 2H), 8.42 (br d, 1H), 3.23 (s, 3H), 2.53 (s, 3H), 1.60 - 1.28 (br m, 9H) |
| E3 | 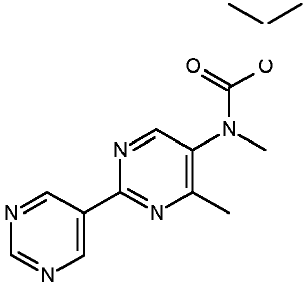 | 9.70 (s, 2H), 9.31 (s, 1H), 8.55 (s, 1H), 3.24 (s, 3H), 2.54 (s, 3H), 1.60-1.35 (br m, 9H) |
| E4 | 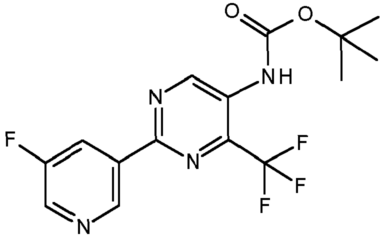 | 9.81 (s, 1H), 9.45 (s, 1H), 8.58 (d, 1H), 8.39 (d, 1H), 6.97 (br s, 1H), 1.58 (m, 9H) |
| E5 | 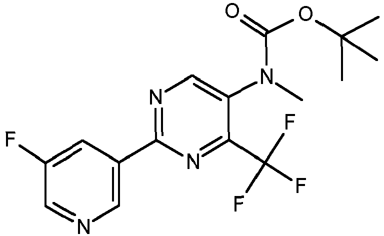 | 9.52 (s, 1H), 8.85 (s, 1H), 8.63 (d, 1H), 8.47 (br d, 1H), 3.25 (s, 3H), 1.38 (s, 9H) |
| E6 | 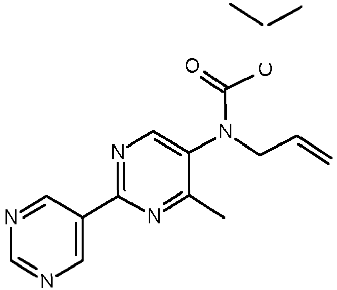 | (CD₃OD) 9.68 (s, 2H), 9.22 (s, 1H), 8.62 (br. s, 1H), 5.92 (br. s, 1H), 5.22-5.07 (br. m, 2H), 4.48-4.07 (br. m, 2H), 2.52 (s, 3H), 1.54-1.47 (br. s, 4H), 1.47-1.22 (br. s, 5H) |
| E7 | 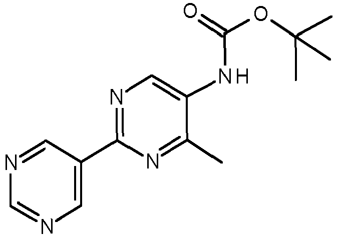 | (CD₃OD) 9.64 (s, 2H), 9.22 (s, 1H), 9.00 (s, 1H), 2.57 (s, 3H), 1.55 (s, 9H) |
| E8 | 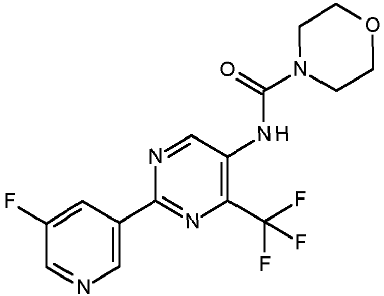 | 9.78 (s, 1H), 9.45 (s, 1H), 8.58 (d, 1H), 8.39 (d, 1H), 6.85 (br s, 1H), 3.82-3.75 (m, 4H), 3.58-3.52 (m, 4H) |
| E9 | 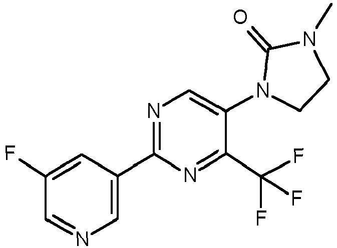 | 9.50 (s, 1H), 9.01 (s, 1H), 8.61 (d, 1H), 8.43 (d, 1H), 3.87-3.80 (m, 2H), 3.64-3.58 (m, 2H), 2.95 (s, 3H) |
| E10 | 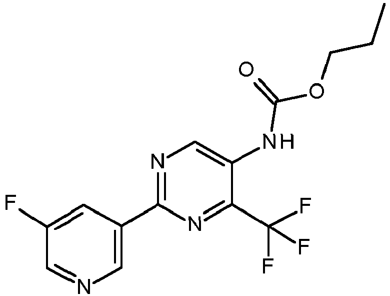 | 9.81 (s, 1H), 9.45 (s, 1H), 8.59 (d, 1H), 8.42-8.36 (m, 1H), 6.99 (br s, 1H), 4.22 (t, 2H), 1.81-1.71 (m, 2H), 1.01 (t, 3H) |
| E11 | 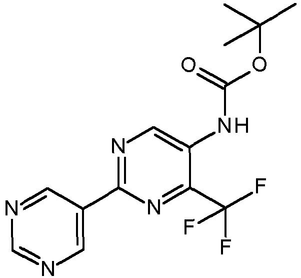 | 9.79 (s, 1H), 9.61 (s, 2H), 9.25 (s, 1H), 6.83 (br s, 1H), 1.50 (s, 9H) |
| E12 | 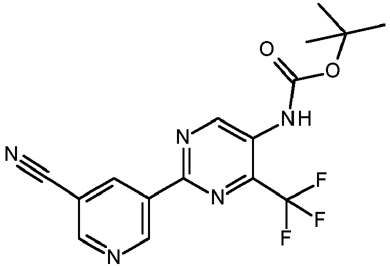 | 9.89 (s, 1H), 9.81 (d, 1H), 8.99-8.94 (m, 2H), 6.92 (br s, 1H), 1.58 (s, 9H) |
| E13 | 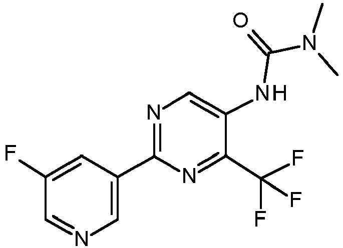 | 9.83 (s, 1H), 9.44 (s, 1H), 8.56 (d, 1H), 8.42-8.35 (m, 1H), 6.90 (br s, 1H), 3.10 (s, 6H) |
| E14 | 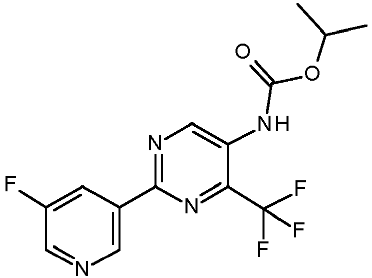 | 9.84 (s, 1H), 9.46 (s, 1H), 8.59 (d, 1H), 8.42-8.37 (m, 1H), 6.95 (br s, 1H), 5.15-5.03 (m, 1H), 1.36 (d, 6H) |
| E15 | 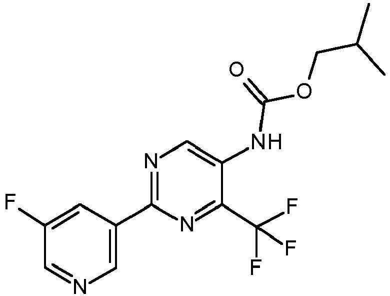 | 9.81 (s, 1H), 9.47 (s, 1H), 8.59 (d, 1H), 8.42-8.38 (m, 1H), 7.01 (br s, 1H), 4.04 (d, 2H), 2.11-1.98 (m, 1H), 1.00 (d, 6H) |
| E17 | 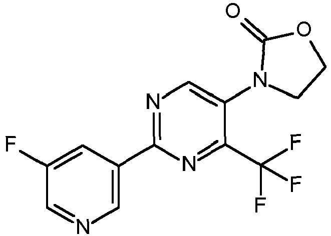 | 9.53 (s, 1H), 9.06 (s, 1H), 8.68 (d, 1H), 8.56-8.49 (m, 1H), 4.65 (t, 2H), 4.08 (t, 2H) |
| E18 | 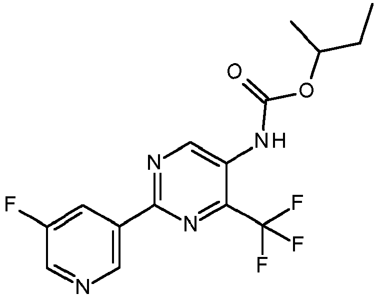 | 9.82 (s, 1H), 9.45 (s, 1H), 8.59 (d, 1H), 8.41-8.37 (m, 1H), 6.95 (br s, 1H), 4.98-4.88 (m, 1H), 1.79-1.60 (m, 2H), 1.32 (d, 3H), 0.98 (t, 3H) |
| E19 | 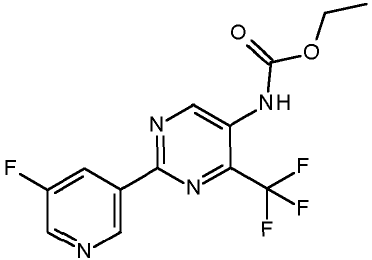 | 9.82 (s, 1H), 9.45 (s, 1H), 8.59 (d, 1H), 8.41-8.38 (m, 1H), 7.00 (br s, 1H), 4.32 (q, 2H), 1.39 (t, 3H) |
| E20 | 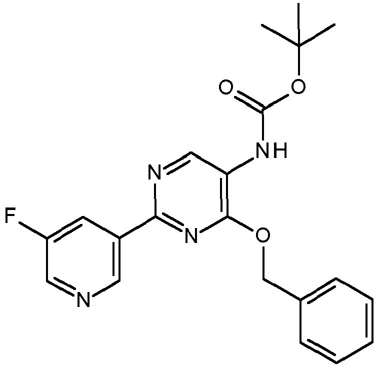 | 9.39-9.36 (m, 1H), 9.29 (br s, 1H), 8.51 (d, 1H), 8.34-8.29 (m, 1H), 7.52-7.48 (m, 2H), 7.48-7.37 (m, 3H), 6.82 (br s, 1H), 5.60 (s, 2H), 1.53 (s, 9H) |
| E21 | 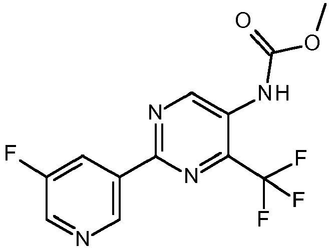 | 9.81 (s, 1H), 9.46 (s, 1H), 8.59 (d, 1H), 8.42-8.38 (m, 1H), 7.03 (br s, 1H), 3.89 (s, 3H) |
| E22 | 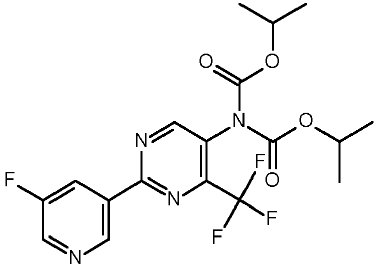 | 9.55 (s, 1H), 8.71 (s, 1H), 8.65 (s, 1H), 8.49 (d, 1H), 5.08 (m, 2H) 1.22 (d, 12H) |

### Physical characterisation

Compounds of the invention were characterised using one or more of the following methods.

### NMR

NMR spectra contained herein were recorded on either a 400MHz Bruker AVANCE III HD equipped with a Bruker SMART probe or a 500MHz Bruker AVANCE III equipped with a Bruker Prodigy probe. Chemical shifts are expressed as ppm downfield from TMS, with an internal reference of either TMS or the residual solvent signals. The following multiplicities are used to describe the peaks: s = singlet, d = doublet, t = triplet, dd = double doublet, m = multiplet. Additionally br. is used to describe a broad signal and app. is used to describe an apparent multiplicity.

### LCMS

LCMS data contained herein consists of the molecular ion [MH+] and the retention time (tr) of the peak recorded on the chromatogram. The following instruments, methods and conditions were used to obtain LCMS data:

### Method A

*Instrumentation:* Waters Acquity UPLC-MS using a Sample Organizer with Sample Manager FTN, H-Class QSM, Column Manager, 2 x Column Manager Aux, Photodiode Array (Wavelength range (nm): 210 to 400, ELSD and SQD 2 equipped with a Waters HSS T3 C18 column (column length 30 mm, internal diameter of column 2.1 mm, particle size 1.8 micron).

*lonisation method:* Electrospray positive and negative: Capillary (kV) 3.00, Cone (V) 30.00, Source Temperature (°C) 500, Cone Gas Flow (L/Hr.) 10, Desolvation Gas Flow (L/Hr.) 1000. Mass range (Da): positive 95 to 800, negative 115 to 800.

The analysis was conducted using a two minute run time, according to the following gradient table at 40°C:

| **Time (mins)** | **Solvent A (%)** | **Solvent B (%)** | **Flow (ml / mn)** |
|---|---|---|---|
| 0.00 | 95.0 | 5.0 | 0.7 |
| 1.75 | 0.0 | 100 | 0.7 |
| 1.76 | 0.0 | 100 | 0.7 |
| 2.0 | 0.0 | 5.0 | 0.7 |
| 2.01 | 95.0 | 5.0 | 0.7 |
| 2.11 | 95.0 | 5.0 | 0.7 |

Solvent A: H₂O with 0.05% TFA
Solvent B: CH₃CN with 0.05% TFA

### Method B (2 min method)

*Instrumentation:* Either (a) Waters Acquity UPLC system with Waters SQD2 single-quad MS detector, Photodiode Array Detector (Absorbance Wavelength: 254 nm, 10 pts/sec, Time Constant: 0.2000 sec), Charged Aerosol Detector (Corona) and Waters CTC 2770 auto-sampler unit (injection volume: 2 microliters, 1 min seal wash); or (b) Waters Acquity UPLC system with Waters QDa single-quad MS detector, Photodiode Array Detector (Absorbance Wavelength: 254 nm, 10 pts/sec, Time Constant: 0.2000 sec), Charged Aerosol Detector (Corona) and Waters CTC 2770 auto-sampler unit (injection volume: 2 microliters, 1 min seal wash).

### LC-Method:

Phenomenex 'Kinetex C18 100A' column (50 mm x 4.6 mm, particle size 2.6 micron),
Flow rate: 2 mL/min at 313K (40 Celsius),
Gradient (Solvent A: H₂O with 0.1% Formic Acid; Solvent B: Acetonitrile with 0.1% Formic Acid):
The analysis was conducted using a two minute run time, according to the following gradient table at 40°C.

| **Time (mins)** | **Solvent A (%)** | **Solvent B (%)** | **Flow (ml / mn)** |
|---|---|---|---|
| Initial | 70.0 | 30.0 | 2.000 |
| 1.20 | 10.0 | 90.0 | 2.000 |
| 1.70 | 10.0 | 90.0 | 2.000 |
| 1.80 | 70.0 | 30.0 | 2.000 |
| 2.00 | 70.0 | 30.0 | 2.000 |
| 2.20 | 70.0 | 30.0 | 2.000 |

### Method C (1 min method)

*Instrumentation:* Either (a) Waters Acquity UPLC system with Waters SQD2 single-quad MS detector, Photodiode Array Detector (Absorbance Wavelength: 254 nm, 10 pts/sec, Time Constant: 0.2000 sec), Charged Aerosol Detector (Corona) and Waters CTC 2770 auto-sampler unit (injection volume: 2 microliters, 1 min seal wash); or (b) Waters Acquity UPLC system with Waters QDa single-quad MS detector, Photodiode Array Detector (Absorbance Wavelength: 254 nm, 10 pts/sec, Time Constant: 0.2000 sec), Charged Aerosol Detector (Corona) and Waters CTC 2770 auto-sampler unit (injection volume: 2 microliters, 1 min seal wash).

### LC-Method:

Phenomenex 'Kinetex C18 100A' column (50 mm x 4.6 mm, particle size 2.6 micron),
Flow rate: 2 mL/min at 313K (40 Celsius),
Gradient (Solvent A: H₂O with 0.1% Formic Acid; Solvent B: Acetonitrile with 0.1% Formic Acid):
The analysis was conducted using a one minute run time, according to the following gradient table at 40°C.

| **Time (mins)** | **Solvent A (%)** | **Solvent B (%)** | **Flow (ml / mn)** |
|---|---|---|---|
| Initial | 60.0 | 40.0 | 2.000 |
| 0.80 | 0.0 | 100.0 | 2.000 |
| 0.95 | 0.0 | 100.0 | 2.000 |
| 1.00 | 60.0 | 40.0 | 2.000 |
| 1.10 | 60.0 | 40.0 | 2.000 |
| 1.25 | 60.0 | 40.0 | 2.000 |

### BIOLOGICIAL EXAMPLES

### B1 Pre-emergence herbicidal activity

Seeds of a variety of test species were sown in standard soil in pots: *Triticum aestivium* (TRZAW), *Avena fatua* (AVEFA), *Alopecurus myosuroides* (ALOMY), *Echinochloa crus-galli* (ECHCG), *Lolium perenne* (LOLPE), *Zea Mays* (ZEAMX), *Abutilon theophrasti* (ABUTH), *Amaranthus retroflexus* (AMARE) and *Setaria faberi* (SETFA). After cultivation for one day (pre-emergence) under controlled conditions in a glasshouse (at 24/16°C, day/night; 14 hours light; 65% humidity), the plants were sprayed with an aqueous spray solution derived from the formulation of the technical active ingredient in acetone / water (50:50) solution containing 0.5% Tween 20 (polyoxyethelyene sorbitan monolaurate, CAS RN 9005-64-5). The test plants were then grown in a glasshouse under controlled conditions (at 24/16°C, day/night; 14 hours light; 65% humidity) and watered twice daily. After 13 days, the test was evaluated (5= total damage to plant; 0 = no damage to plant). Results are shown in Tables B1a and B1b.

### Tables B1a and B1b Control of weed species by compound of Formula (I) after pre-emergence application

**Table B1a: Test 1a**

| **Compound ID** | **Rate (g/ha)** | **AMARE** | **ABUTH** | **SETFA** | **LOLPE** | **ECHCG** | **ZEAMX** |
|---|---|---|---|---|---|---|---|
| E1 | 1000 | 0 | 0 | 4 | 1 | 4 | 5 |
| E2 | 1000 | 0 | 0 | 4 | 0 | 2 | 4 |

**Table B1b: Test 1b**

| **Compound ID** | **Rate (g/ha)** | **ECHCG** | **LOLPE** | **SETFA** | **AVEFA** | **ALOMY** | **TRAZW** |
|---|---|---|---|---|---|---|---|
| E3 | 1000 | 4 | 1 | 4 | 4 | 1 | 0 |
| E4 | 1000 | 4 | 1 | 4 | 4 | 0 | 0 |
| E5 | 1000 | 3 | 1 | 5 | 3 | 0 | 0 |
| E6 | 1000 | 3 | 1 | 5 | 1 | 0 | 0 |
| E7 | 1000 | 5 | 1 | 4 | 2 | 0 | 0 |
| E8 | 1000 | 3 | 1 | 4 | 2 | 0 | 0 |
| E9 | 1000 | 1 | 1 | 2 | 1 | 0 | 0 |
| E10 | 1000 | 4 | 1 | NT | 2 | 0 | 0 |
| E11 | 1000 | 3 | 1 | 4 | 2 | 1 | 0 |
| E12 | 1000 | 3 | 0 | 4 | 0 | 0 | 0 |
| E13 | 1000 | 3 | 1 | 4 | 1 | 0 | 0 |
| E14 | 1000 | 3 | 0 | 5 | 1 | 0 | 0 |
| E15 | 1000 | 5 | 1 | 5 | 2 | 0 | 0 |
| E17 | 1000 | 3 | 0 | 4 | 0 | 0 | 0 |
| E18 | 1000 | 2 | 0 | 5 | 1 | 0 | 1 |
| E19 | 1000 | 3 | 1 | 4 | 0 | 0 | 0 |
| E20 | 1000 | 0 | 0 | 0 | 0 | 0 | 0 |
| E21 | 1000 | 3 | 0 | 4 | 0 | 1 | 1 |
| E22 | 1000 | 3 | 0 | 4 | 0 | 0 | 0 |

Compounds that score 4 or 5 on one or more plant species are particularly preferred.

### B2 Post-emergence herbicidal activity

Seeds of a variety of test species were sown in standard soil in pots: *Triticum aestivium* (TRZAW), *Avena fatua* (AVEFA), *Alopecurus myosuroides* (ALOMY), *Echinochloa crus-galli* (ECHCG), *Lolium perenne* (LOLPE), *Zea Mays* (ZEAMX), *Abutilon theophrasti* (ABUTH), *Amaranthus retroflexus* (AMARE) and *Setaria faberi* (SETFA). After 8 days cultivation (post-emergence) under controlled conditions in a glasshouse (at 24/16°C, day/night; 14 hours light; 65% humidity), the plants were sprayed with an aqueous spray solution derived from the formulation of the technical active ingredient in acetone / water (50:50) solution containing 0.5% Tween 20 (polyoxyethelyene sorbitan monolaurate, CAS RN 9005-64-5). The test plants were then grown in a glasshouse under controlled conditions (at 24/16°C, day/night; 14 hours light; 65% humidity) and watered twice daily. After 13 days, the test was evaluated (5 = total damage to plant; 0 = no damage to plant). Results are shown in Tables B2a and B2b.

### Tables B2a and B2b Control of weed species by compound of Formula (I) after post-emergence application

**Table B2a Test 2a**

| **Compound ID** | **Rate (g/ha)** | **AMARE** | **ABUTH** | **SETFA** | **LOLPE** | **ECHCG** | **ZEAMX** |
|---|---|---|---|---|---|---|---|
| E1 | 1000 | 2 | 1 | 5 | 2 | 4 | 5 |
| E2 | 1000 | 0 | 1 | 5 | 2 | 5 | 5 |

**Table B2b: Test 2b**

| **Compound ID** | **Rate (g/ha)** | **ECHCG** | **LOLPE** | **SETFA** | **AVEFA** | **ALOMY** | **TRAZW** |
|---|---|---|---|---|---|---|---|
| E3 | 1000 | 4 | 3 | 5 | 4 | 2 | 1 |
| E4 | 1000 | 4 | 3 | 5 | 4 | 1 | 1 |
| E5 | 1000 | 4 | 2 | 5 | 4 | 0 | 0 |
| E6 | 1000 | 3 | 1 | 5 | 2 | 1 | 1 |
| E7 | 1000 | 4 | 2 | 4 | 3 | 0 | 0 |
| E8 | 1000 | 4 | 3 | 5 | 3 | 1 | 1 |
| E9 | 1000 | 4 | 1 | 3 | 2 | 0 | 1 |
| E10 | 1000 | 4 | 1 | 5 | 3 | 0 | 1 |
| E11 | 1000 | 4 | 2 | 4 | 3 | 0 | 1 |
| E12 | 1000 | 2 | 1 | 5 | 2 | 0 | 0 |
| E13 | 1000 | 4 | 3 | 5 | 3 | 1 | 2 |
| E14 | 1000 | 4 | 3 | 5 | 3 | 0 | 1 |
| E15 | 1000 | 5 | 3 | 5 | 4 | 1 | 2 |
| E17 | 1000 | 3 | 1 | 4 | 2 | 0 | 1 |
| E18 | 1000 | 4 | 1 | 5 | 1 | 0 | 0 |
| E19 | 1000 | 5 | 3 | 5 | 3 | 0 | 1 |
| E20 | 1000 | 0 | 0 | 1 | 0 | 0 | 0 |
| E21 | 1000 | 5 | 2 | 4 | 4 | 1 | 1 |
| E22 | 1000 | 5 | 4 | 1 | 0 | 0 | 4 |

Compounds which score 4 or 5 on one or more plant species are particularly preferred.

## Claims

1. A compound of Formula (I) 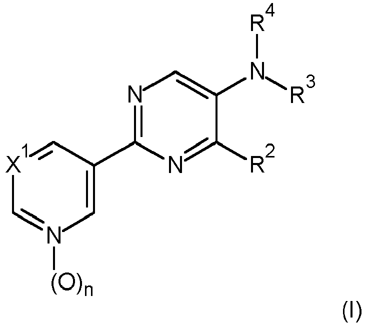 or a salt or N-oxide thereof, wherein,
X¹ is N or CR¹;
R¹ is selected from the group consisting of hydrogen, halogen, cyano, C₁-C₆alkyl, C₃-C₆cycloalkyl, C₂-C₆alkenyl, C₂-C₆alkynyl, C₁-C₆alkoxy, -C(O)OC₁-C₆alkyl, - S(O)ₚC₁-C₆alkyl, NR⁶R⁷, C₁-C₆haloalkoxy and C₁-C₆haloalkyl;
R² is selected from the group consisting of halogen, cyano, nitro, C₁-C₆alkyl, C₁-C₆haloalkyl, C₂-C₆alkenyl, C₂-C₆alkynyl, C₃-C₆cycloalkyl, -C(O)OC₁-C₆alkyl, - S(O)ₚ(C₁-C₆alkyl), C₁-C₆alkoxy, C₁-C₆haloalkoxy, phenyl, and benzyloxy;
R³ is -C(O)X²R¹²;
X² is O or NR¹⁰;
R⁴ is selected from the group consisting of hydrogen, C₁-C₆alkyl, C₁-C₆alkoxy, C₁-C₆haloalkyl, C₁-C₆haloalkoxy, C₃-C₆cycloalkyl, C₃-C₆alkenyl, C₃-C₆alkynyl, - C(O)R⁹-(CR^{a}R^{b})_{q}R⁵, -C(O)X³R¹³;
when X² is O, R¹² is selected from the group consisting of C₁-C₆alkyl, CᵣalkoxyCₛalkyl, C₁-C₆haloalkyl, CᵣalkoxyCₛhaloalkyl, CᵣalkylthioCₛalkyl, C₂-C₆alkenyl, C₂-C₆alkynyl, and -(CR^{a}R^{b})_{q}R¹¹, or R⁴ and R¹² together with the heteroatoms to which they are joined form a 5-7 membered ring system optionally optionally containing 1 additional heteroatom selected from S, O and N, wherein when said additional heteroatom is sulphur it is in the form S(O)ₚ, when X² is NR¹⁰, R¹² is selected from the group consisting of hydrogen, C₁-C₆alkyl, C₁-C₆alkoxy, C₁-C₆haloalkyl, C₁-C₆haloalkoxy, CᵣalkylthioCₛalkyl, C₂-C₆alkenyl, C₂-C₆alkynyl, and -(CR^{a}R^{b})_{q}R¹¹; or R¹⁰ and R¹² together with the nitrogen atom to which they are both joined, can form a 5-, 6-, or 7-memberered ring, optionally containing 1 to 3 additional heteroatoms each independently selected from O, N or S, wherein when said ring contains a ring sulphur, said ring sulphur is in the form S(O)ₚ; or R⁴ and R¹⁰ together with the atoms to which they are joined form a 5-7 membered ring system optionally comprising from 1 or 2 additional heteroatoms independently selected from S, O and N and wherein when said ring system contains a ring sulphur, said ring sulphur is in the form S(O)ₚ; or,
R¹⁰ is independently selected from the group consisting of hydrogen, C₁-C₆alkyl, C₃-C₆cycloalkyl;
when R⁴ is -C(O)X³R¹³, X³ is O or NR¹⁴;
when X³ is O, R¹³ is selected from the group consisting of C₁-C₆alkyl, CᵣalkoxyCₛalkyl, C₁-C₆haloalkyl, CᵣalkoxyCₛhaloalkyl, CᵣalkylthioCₛalkyl, C₂-C₆alkenyl, C₂-C₆alkynyl, and -(CR^{a}R^{b})_{q}R¹¹, or R³ and R¹³ together with the heteroatoms to which they are joined form a 5-7 membered ring system optionally optionally containing 1 additional heteroatom selected from S, O and N, wherein when said additional heteroatom is sulphur it is in the form S(O)ₚ, or
when X³ is NR¹⁴, R¹³ is selected from the group consisting of hydrogen, C₁-C₆alkyl, C₁-C₆alkoxy, C₁-C₆haloalkyl, C₁-C₆haloalkoxy, CᵣalkylthioCₛalkyl, C₂-C₆alkenyl, C₂-C₆alkynyl, and -(CR^{a}R^{b})_{q}R¹¹; or R¹⁴ and R¹³ together with the nitrogen atom to which they are both joined, can form a 5-, 6-, or 7-memberered ring, optionally containing 1 or 2 additional heteroatoms each independently selected from O, N or S, wherein when said ring contains a ring sulphur, said ring sulphur is in the form S(O)ₚ;
R^{a} is hydrogen or C₁-C₂ alkyl;
R^{b} is hydrogen or C₁-C₂ alkyl;
R⁵ is cyano, -C(O)OC₁-C₆alkyl, -C₃-C₆cycloalkyl, -aryl or ―heteroaryl wherein said aryl and heteroaryl are optionally substituted by 1 to 3 independent R⁸;
R⁶ and R⁷ are independently selected from the group consisting of hydrogen, C₁-C₆alkyl, and ―C(O)OC₁-C₄alkyl;
each R⁸ is independently selected from the group consisting of halogen, C₁-C₆alkyl and C₁-C₆alkoxy-, C₁-C₆ haloalkyl, C₁-C₆ haloalkoxy-, cyano and S(O)ₚ(C₁-C₆alkyl);
R⁹ is selected from the group consisting of hydrogen, C₁-C₆alkyl, C₁-C₆alkoxy, C₁-C₆haloalkyl, C₁-C₆haloalkoxy, C₂-C₆alkenyl, C₂-C₆alkynyl, and -(CR^{a}R^{b})_{q}R¹¹;
R¹¹ is cyano, -C₃-C₆cycloalkyl, or an ―aryl, -heteroaryl or -heterocyclyl ring, wherein said ring is optionally substituted by 1 to 3 independent R⁸, and wherein when said ring contains a ring sulphur, said ring sulphur is in the form S(O)ₚ;
n is 0 or 1;
p is 0, 1, or 2;
q is 0, 1, 2, 3, 4, 5 or 6;
r is 1, 2, 3, 4, or 5, s is 1, 2, 3, 4, or 5, and the sum of r+s is less than or equal to 6.

2. The compound of Formula (I) according to claim 1, wherein X¹ is N.

3. The compound of Formula (I) according to claim 1, wherein X¹ is CR¹ and R¹ is selected from the group consisting of hydrogen, cyano, halogen, C₁-C₃alkyl, C₃-C₄alkynyl, C₁-C₃alkoxy, C₁-C₃haloalkyl, C₁-C₃haloalkoxy, NR⁶R⁷, and C₁-C₃thioalkyl.

4. The compound of Formula (I) according to any one of the preceding claims, wherein R² is halogen, cyano, C₁-C₆alkyl,C₁-C₆haloalkyl, C(O)OC₁-C₆alkyl, phenyl, and benzyloxy.

5. The compound of Formula (I) according to any one of the preceding claims, wherein X² is O.

6. The compound of Formula (I) according to claim 5, wherein R¹² is selected from the group consisting of: hydrogen, C₁-C₆alkyl,CᵣalkoxyCₛalkyl, C₁-C₆haloalkyl, CᵣalkoxyCₛhaloalkyl, CᵣalkylthioCₛalkyl, C₂-C₆alkenyl, C₂-C₆alkynyl, and -(CR^{a}R^{b})_{q}R¹¹

7. The compound according to claim 5, wherein R⁴ and R¹² together with the atoms qto which they are joined form a 5-7 membered ring system optionally containing 1 or 2 additional heteroatoms independently selected from S in the form S(O)ₚ, O and N.

8. The compound of formula (I) according to any one of claims 1 to 4, wherein X² is NR¹⁰.

9. The compound of formula (I) according to claim 8, wherein R¹⁰ is:hydrogen or C₁-C₆alkyl.

10. The compound of formula (I) according to claim 8, wherein R¹⁰ together with R⁴ and the atoms to which R¹⁰ and R⁴ are joined form a 5-7 membered ring system optionally containing from 1 to 3 additional heteroatoms independently selected from S in the form of S(O)ₚ, O and N.

11. The compound of formula (I) according to claim 8, wherein R¹⁰ together with R¹² and the nitrogen atom to which R¹⁰ and R¹² are joined form a 5-7 membered ring system optionally containing from 1 to 3 additional heteroatoms independently selected from S in the form of S(O)ₚ, O and N.

12. The compound according to claim 8, wherein R¹⁰ is hydrogen or C₁-C₆alkyl, and R¹² is C₁-C₄alkyl, C₁-C₃alkoxy, -(CH₂)₃SCH₃, C₁-C₃haloalkyl, C₃-C₆alkynyl, or (CR^{a}R^{b})_{q}R¹¹.

13. The compound of Formula (I) according to any one of claims 1 to 6, 8, 9, 11 or 12, wherein R⁴ is selected from the group consisting of hydrogen, methyl, ethyl, allyl, but-2-ynyl, C(O)R⁹ and -(CH₂)_{q}R⁵.

14. The compound of Formula (I) acoording to clim 13, wherin R⁹ is C₁-C₆alkoxy.

15. A herbicidal composition comprising a compound according to any one of the previous claims and an agriculturally acceptable formulation adjuvant.

16. The herbicidal composition according to claim 15, further comprising at least one additional pesticide.

17. The herbicidal composition according to claim 16, wherein the additional pesticide is a herbicide or herbicide safener.

18. A method of controlling weeds at a locus comprising application to the locus of a weed controlling amount of a composition according to any one of claims 15 to 17.

19. Use of a compound of Formula (I) as defined in any one of claims 1 to 14, as a herbicide.

## Patentansprüche

1. Verbindung der Formel (I) 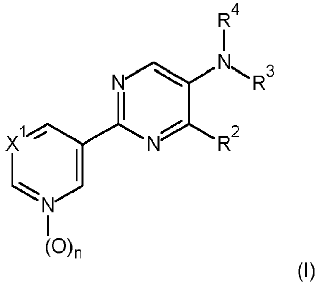 oder ein Salz oder N-Oxid davon, wobei
X¹ für N oder CR¹ steht;
R¹ aus der Gruppe bestehend aus Wasserstoff, Halogen, Cyano, C₁-C₆-Alkyl, C₃-C₆-Cycloalkyl, C₂-C₆-Alkenyl, C₂-C₆-Alkinyl, C₁-C₆-Alkoxy, -C(O)O-C₁-C₆-Alkyl, -S(O)ₚ-C₁-C₆-Alkyl, NR⁶R⁷, C₁-C₆-Halogenalkoxy und C₁-C₆-Halogenalkyl ausgewählt ist;
R² aus der Gruppe bestehend aus Halogen, Cyano, Nitro, C₁-C₆-Alkyl, C₁-C₆-Halogenalkyl, C₂-C₆-Alkenyl, C₂-C₆-Alkinyl, C₃-C₆-Cycloalkyl, -C(O)O-C₁-C₆-Alkyl, -S(O)ₚ-(C₁-C₆-Alkyl) , C₁-C₆-Alkoxy, C₁-C₆-Halogenalkoxy, Phenyl und Benzyloxy ausgewählt ist;
R³ für -C(O)X²R¹² steht;
X² für O oder NR¹⁰ steht;
R⁴ aus der Gruppe bestehend aus Wasserstoff, C₁-C₆-Alkyl, C₁-C₆-Alkoxy, C₁-C₆-Halogenalkyl, C₁-C₆-Halogenalkoxy, C₃-C₆-Cycloalkyl, C₃-C₆-Alkenyl, C₃-C₆-Alkinyl, -C(O)R⁹, -(CR^{a}R^{b})_{q}R⁵, -C(O)X³R¹³ ausgewählt ist;
dann, wenn X² für O steht, R¹² aus der Gruppe bestehend aus C₁-C₆-Alkyl, Cᵣ-Alkoxy-Cₛ-alkyl, C₁-C₆-Halogenalkyl, Cᵣ-Alkoxy-Cₛ-halogenalkyl, Cᵣ-Alkylthio-Cₛ-alkyl, C₂-C₆-Alkenyl, C₂-C₆-Alkinyl und -(CR^{a}R^{b})_{q}R¹¹ ausgewählt ist oder R⁴ und R¹² zusammen mit den Heteroatomen, an die sie gebunden sind, ein 5-7-gliedriges Ringsystem bilden, das gegebenenfalls 1 zusätzliches Heteroatom, das aus S, O und N ausgewählt ist, enthält, wobei das zusätzliche Heteroatom dann, wenn es sich dabei um Schwefel handelt, in der Form S(O)ₚ vorliegt;
dann, wenn X² für NR¹⁰ steht, R¹² aus der Gruppe bestehend aus Wasserstoff, C₁-C₆-Alkyl, C₁-C₆-Alkoxy, C₁-C₆-Halogenalkyl, C₁-C₆-Halogen-alkoxy, Cᵣ-Alkylthio-Cₛ-alkyl, C₂-C₆-Alkenyl, C₂-C₆-Alkinyl und -(CR^{a}R^{b})_{q}R¹¹ ausgewählt ist oder R¹⁰ und R¹² zusammen mit dem Stickstoffatom, an das sie beide gebunden sind, einen 5-, 6- oder 7-gliedrigen Ring bilden können, der gegebenenfalls 1 bis 3 zusätzliche Heteroatome, die jeweils unabhängig aus O, N oder S ausgewählt sind, enthält, wobei dann, wenn der Ring einen Ringschwefel enthält, der Ringschwefel in der Form S(O)ₚ vorliegt; oder R⁴ und R¹⁰ zusammen mit den Atomen, an die sie gebunden sind, ein 5-7-gliedriges Ringsystem bilden, das gegebenenfalls 1 oder 2 zusätzliche Heteroatome, die unabhängig aus S, O und N ausgewählt sind, enthält, und wobei dann, wenn das Ringsystem einen Ringschwefel enthält, der Ringschwefel in der Form S(O)ₚ vorliegt; oder
R¹⁰ unabhängig aus der Gruppe bestehend aus Wasserstoff, C₁-C₆-Alkyl, C₃-C₆-Cycloalkyl ausgewählt ist;
dann, wenn R⁴ für -C(O)X³R¹³ steht, X³ für O oder NR¹⁴ steht;
dann, wenn X³ für O steht, R¹³ aus der Gruppe bestehend aus C₁-C₆-Alkyl, Cᵣ-Alkoxy-Cₛ-alkyl, C₁-C₆-Halogenalkyl, Cᵣ-Alkoxy-Cₛ-halogenalkyl, Cᵣ-Alkylthio-Cₛ-alkyl, C₂-C₆-Alkenyl, C₂-C₆-Alkinyl und -(CR^{a}R^{b})_{q}R¹¹ ausgewählt ist oder R³ und R¹³ zusammen mit den Heteroatomen, an die sie gebunden sind, ein 5-7-gliedriges Ringsystem bilden, das gegebenenfalls 1 zusätzliches Heteroatom, das aus S, O und N ausgewählt ist, enthält, wobei das zusätzliche Heteroatom dann, wenn es sich dabei um Schwefel handelt, in der Form S(O)ₚ vorliegt, oder dann, wenn X³ für NR¹⁴ steht, R¹³ aus der Gruppe bestehend aus Wasserstoff, C₁-C₆-Alkyl, C₁-C₆-Alkoxy, C₁-C₆-Halogenalkyl, C₁-C₆-Halogen-alkoxy, Cᵣ-Alkylthio-Cₛ-alkyl, C₂-C₆-Alkenyl, C₂-C₆-Alkinyl und -(CR^{a}R^{b})_{q}R¹¹ ausgewählt ist oder R¹⁴ und R¹³ zusammen mit dem Stickstoffatom, an das sie beide gebunden sind, einen 5-, 6- oder 7-gliedrigen Ring bilden können, der gegebenenfalls 1 oder 2 zusätzliche Heteroatome, die jeweils unabhängig aus S, O und N ausgewählt sind, enthält, wobei dann, wenn der Ring einen Ringschwefel enthält, der Ringschwefel in der Form S(O)ₚ vorliegt;
R^{a} für Wasserstoff oder C₁-C₂-Alkyl steht;
R^{b} für Wasserstoff oder C₁-C₂-Alkyl steht;
R⁵ für Cyano, -C(O)O-C₁-C₆-Alkyl, -C₃-C₆-Cycloalkyl, -Aryl oder -Heteroaryl steht, wobei das Aryl und Heteroaryl gegebenenfalls durch 1 bis 3 unabhängige R⁸ substituiert sind;
R⁶ und R⁷ unabhängig aus der Gruppe bestehend aus Wasserstoff, C₁-C₆-Alkyl und -C(O)O-C₁-C₄-Alkyl ausgewählt sind;
R⁸ jeweils unabhängig aus der Gruppe bestehend aus Halogen, C₁-C₆-Alkyl und C₁-C₆-Alkoxy-, C₁-C₆-Halogenalkyl, C₁-C₆-Halogenalkoxy-, Cyano und S(O)ₚ(C₁-C₆)-Alkyl ausgewählt ist;
R⁹ aus der Gruppe bestehend aus Wasserstoff, C₁-C₆-Alkyl, C₁-C₆-Alkoxy, C₁-C₆-Halogenalkyl, C₁-C₆-Halogenalkoxy, C₂-C₆-Alkenyl, C₂-C₆-Alkinyl und -(CR^{a}R^{b})_{q}R¹¹ ausgewählt ist;
R¹¹ für Cyano, -C₃-C₆-Cycloalkyl oder einen -Aryl-, -Heteroaryl- oder -Heterocyclylring steht, wobei der Ring gegebenenfalls durch 1 bis 3 unabhängige R⁸ substituiert ist und wobei dann, wenn der Ring einen Ringschwefel enthält, der Ringschwefel in der Form S(O)ₚ vorliegt;
n für 0 oder 1 steht;
p für 0, 1 oder 2 steht;
q für 0, 1, 2, 3, 4, 5 oder 6 steht;
r für 1, 2, 3, 4 oder 5 steht, s für 1, 2, 3, 4 oder 5 steht und die Summe von r + s kleiner als oder gleich 6 ist.

2. Verbindung der Formel (I) nach Anspruch 1, wobei X¹ für N steht.

3. Verbindung der Formel (I) nach Anspruch 1, wobei X¹ für CR¹ steht und R¹ aus der Gruppe bestehend aus Wasserstoff, Cyano, Halogen, C₁-C₃-Alkyl, C₃-C₄-Alkinyl, C₁-C₃-Alkoxy, C₁-C₃-Halogenalkyl, C₁-C₃-Halogenalkoxy, NR⁶R⁷ und C₁-C₃-Thioalkyl ausgewählt ist.

4. Verbindung der Formel (I) nach einem der vorhergehenden Ansprüche, wobei R² für Halogen, Cyano, C₁-C₆-Alkyl,C₁-C₆-Halogenalkyl, C(O)O-C₁-C₆-Alkyl, Phenyl und Benzyloxy ausgewählt ist

5. Verbindung der Formel (I) nach einem der vorhergehenden Ansprüche, wobei X² für O steht.

6. Verbindung der Formel (I) nach Anspruch 5, wobei R¹² aus der Gruppe bestehend aus Wasserstoff, C₁-C₆-Alkyl,Cᵣ-Alkoxy-Cₛ-alkyl, C₁-C₆-Halogenalkyl, -Cᵣ-Alkoxy-Cₛ-halogenalkyl, Cᵣ-Alkylthio-Cₛ-alkyl, C₂-C₆-Alkenyl, C₂-C₆-Alkinyl und -(CR^{a}R^{b})_{q}R¹¹ ausgewählt ist.

7. Verbindung nach Anspruch 5, wobei R⁴ und R¹² zusammen mit den Atomen, an die sie gebunden sind, ein 5-7-gliedriges Ringsystem bilden, das gegebenenfalls 1 oder 2 zusätzliche Heteroatome, die unabhängig aus S in der Form S(O)ₚ, O und N ausgewählt sind, enthält.

8. Verbindung der Formel (I) nach einem der Ansprüche 1 bis 4, wobei X² für NR¹⁰ steht.

9. Verbindung der Formel (I) nach Anspruch 8, wobei R¹⁰ für Wasserstoff oder C₁-C₆-Alkyl steht.

10. Verbindung der Formel (I) nach Anspruch 8, wobei R¹⁰ zusammen mit R⁴ und den Atomen, an die R¹⁰ und R⁴ gebunden sind, ein 5-7-gliedriges Ringsystem bilden, das gegebenenfalls 1 bis 3 zusätzliche Heteroatome, die unabhängig aus S in der Form S(O)ₚ, O und N ausgewählt sind, enthält.

11. Verbindung der Formel (I) nach Anspruch 8, wobei R¹⁰ zusammen mit R¹² und dem Stickstoffatom, an das R¹⁰ und R¹² gebunden sind, ein 5-7-gliedriges Ringsystem bilden, das gegebenenfalls 1 bis 3 zusätzliche Heteroatome, die unabhängig aus S in der Form S(O)ₚ, O und N ausgewählt sind, enthält.

12. Verbindung nach Anspruch 8, wobei R¹⁰ für Wasserstoff oder C₁-C₆-Alkyl steht und R¹² für C₁-C₄-Alkyl, C₁-C₃-Alkoxy, -(CH₂)₃SCH₃, C₁-C₃-Halogenalkyl, C₃-C₆-Alkinyl oder -(CR^{a}R^{b})_{q}R¹¹ steht.

13. Verbindung der Formel (I) nach einem der Ansprüche 1 bis 6, 8, 9, 11 oder 12, wobei R⁴ aus der Gruppe bestehend aus Wasserstoff, Methyl, Ethyl, Allyl, But-2-inyl, C(O)R⁹ und -(CH₂)_{q}R⁵ ausgewählt ist.

14. Verbindung der Formel (I) nach Anspruch 13, wobei R⁹ für C₁-C₆-Alkoxy steht.

15. Herbizide Zusammensetzung, umfassend eine Verbindung nach einem der vorhergehenden Ansprüche und ein landwirtschaftlich unbedenkliches Formulierungshilfsmittel.

16. Herbizide Zusammensetzung nach Anspruch 15, ferner umfassend mindestens ein zusätzliches Pestizid.

17. Herbizide Zusammensetzung nach Anspruch 16, wobei es sich bei dem zusätzlichen Pestizid um ein Herbizid oder einen Herbizid-Safener handelt.

18. Verfahren zur Bekämpfung von Unkräutern an einem Standort, bei dem man eine unkrautbekämpfende Menge einer Zusammensetzung nach einem der Ansprüche 15 bis 17 auf den Standort ausbringt.

19. Verwendung einer Verbindung der Formel (I) gemäß einem der Ansprüche 1 bis 14 als Herbizid.

## Revendications

1. Composé de Formule (I) 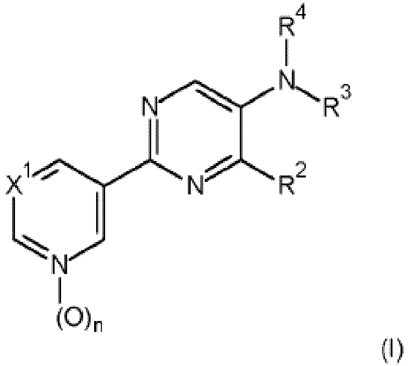 ou sel ou N-oxyde correspondant,
X¹ étant N ou CR¹ ;
R¹ étant choisi dans le groupe constitué par hydrogène, halogène, cyano, C₁-C₆alkyle, C₃-C₆cycloalkyle, C₂-C₆alcényle, C₂-C₆alcynyle, C₁-C₆alcoxy, -C(O)OC₁-C₆alkyle, -S(O)ₚC₁-C₆alkyle, NR⁶R⁷, C₁-C₆halogénoalcoxy et C₁-C₆halogénoalkyle ; R² étant choisi dans le groupe constitué par halogène, cyano, nitro, C₁-C₆alkyle, C₁-C₆halogénoalkyle, C₂-C₆alcényle, C₂-C₆alcynyle, C₃-C₆cycloalkyle, -C(O)OC₁-C₆alkyle, -S(O)ₚ(C₁-C₆alkyle) , C₁-C₆alcoxy, C₁-C₆halogénoalcoxy, phényle, et benzyloxy ;
R³ étant -C(O)X²R¹² ;
X² étant O ou NR¹⁰ ;
R⁴ étant choisi dans le groupe constitué par hydrogène, C₁-C₆alkyle, C₁-C₆alcoxy, C₁-C₆halogénoalkyle, C₁-C₆halogénoalcoxy,C₃-C₆cycloalkyle, C₃-C₆alcényle, C₃-C₆alcynyle, - C(O)R⁹ -(CR^{a}R^{b})R⁵, -C(O)X³R¹³ ;
lorsque X² est O, R¹² est choisi dans le groupe constitué par C₁-C₆alkyle, CᵣalcoxyCₛalkyle, C₁-C₆halogénoalkyle, CᵣalcoxyCₛhalogénoalkyle, CᵣalkylthioCₛalkyle, C₂-C₆alcényle, C₂-C₆alcynyle, et -(CR^{a}R^{b})_{q}R¹¹, ou R⁴ et R¹² conjointement avec les hétéroatomes auxquels ils sont joints formant un système cyclique à 5 à 7 chaînons contenant éventuellement 1 hétéroatome supplémentaire choisi parmi S, O et N, lorsque ledit hétéroatome supplémentaire est un soufre, il est sous la forme de S(O)ₚ,
lorsque X² est NR¹⁰, R¹² est choisi dans le groupe constitué par hydrogène, C₁-C₆alkyle, C₁-C₆alcoxy, C₁-C₆halogénoalkyle, C₁-C₆halogénoalcoxy, CᵣalkylthioCₛalkyle, C₂-C₆alcényle, C₂-C₆alcynyle, et -(CR^{a}R^{b})_{q}R¹¹ ; ou R¹⁰ et R¹² conjointement avec l'atome d'azote auquel ils sont tous les deux joints, pouvant former un cycle à 5, 6 ou 7 chaînons, contenant éventuellement 1 à 3 hétéroatomes supplémentaires chacun indépendamment choisi parmi O, N et S, lorsque ledit cycle contient un soufre de cycle, ledit soufre de cycle est sous la forme de S(O)ₚ ; ou R⁴ et R¹⁰ conjointement avec les atomes auxquels ils sont joints, formant un système cyclique à 5 à 7 chaînons comprenant éventuellement de 1 à 2 hétéroatomes supplémentaires indépendamment choisis parmi S, O et N et lorsque ledit système cyclique contient un soufre de cycle, ledit soufre de cycle est sous la forme de S(O)ₚ ; ou,
R¹⁰ étant indépendamment choisi dans le groupe constitué par hydrogène, C₁-C₆alkyle, C₃-C₆ cycloalkyle ;
lorsque R⁴ est -C(O)X³R¹³, X³ est O ou NR¹⁴ ;
lorsque X³ est O, R¹³ est choisi dans le groupe constitué par C₁-C₆alkyle, CᵣalcoxyCₛalkyle, C₁-C₆halogénoalkyle, CᵣalcoxyCₛhalogénoalkyle, CᵣalkylthioCₛalkyle, C₂-C₆alcényle, C₂-C₆alcynyle, et -(CR^{a}R^{b})_{q}R¹¹, ou R³ et R¹³ conjointement avec les hétéroatomes auxquels ils sont joints formant un système cyclique à 5 à 7 chaînons contenant éventuellement 1 hétéroatome supplémentaire choisi parmi S, O et N, lorsque ledit hétéroatome supplémentaire est un soufre, il est sous la forme de S(O)ₚ, ou
lorsque X³ est NR¹⁴, R¹³ est choisi dans le groupe constitué par hydrogène, C₁-C₆alkyle, C₁-C₆alcoxy, C₁-C₆halogénoalkyle, C₁-C₆halogénoalcoxy, CᵣalkylthioCₛalkyle, C₂-C₆alcényle, C₂-C₆alcynyle, et -(CR^{a}R^{b})_{q}R¹¹ ; ou R¹⁴ et R¹³ conjointement avec l'atome d'azote auquel ils sont tous les deux joints, pouvant former un cycle à 5, 6 ou 7 chaînons, contenant éventuellement 1 ou 2 hétéroatomes supplémentaires chacun indépendamment choisi parmi O, N et S, lorsque ledit cycle contient un soufre de cycle, ledit soufre de cycle est sous la forme de S(O)ₚ ;
R^{a} étant hydrogène ou C₁-C₂ alkyle ;
R^{b} étant hydrogène ou C₁-C₂ alkyle ;
R⁵ étant cyano, -C(O)OC₁-C₆alkyle, C₃-C₆cycloalkyle, -aryle ou -hétéroaryle, lesdits aryle et hétéroaryle étant éventuellement substitués par 1 à 3 R⁸ indépendants ;
R⁶ et R⁷ étant indépendamment choisis dans le groupe constitué par hydrogène, C₁-C₆alkyle, et - C(O)OC₁-C₄alkyle ;
chaque R⁸ étant indépendamment choisi dans le groupe constitué par halogène, C₁-C₆alkyle et C₁-C₆alcoxy-, C₁-C₆halogénoalkyle, C₁-C₆halogénoalcoxy-, cyano et S(O)ₚ(C₁-C₆alkyle) ;
R⁹ étant choisi dans le groupe constitué par hydrogène, C₁-C₆alkyle, C₁-C₆alcoxy, C₁-C₆halogénoalkyle, C₁-C₆halogénoalcoxy,C₂-C₆alcényle, C₂-C₆alcynyle, et -(CR^{a}R^{b})_{q}R¹¹ ;
R¹¹ étant cyano, -C₃-C₆cycloalkyle, ou un cycle - aryle, -hétéroaryle ou -hétérocyclyle, ledit cycle étant éventuellement substitué par 1 à 3 R⁸ indépendants, et lorsque ledit cycle contient un soufre de cycle, ledit soufre de cycle est sous la forme de S(O)ₚ ;
n étant 0 ou 1 ;
p étant 0, 1, ou 2 ;
q étant 0, 1, 2, 3, 4, 5 ou 6 ;
r étant 1, 2, 3, 4, ou 5, s étant 1, 2, 3, 4, ou 5, et la somme de r + s étant inférieure ou égale à 6.

2. Composé de Formule (I) selon la revendication 1, X¹ étant N.

3. Composé de Formule (I) selon la revendication 1, X¹ étant CR¹ et R¹ étant choisi dans le groupe constitué par hydrogène, cyano, halogène, C₁-C₃alkyle, C₃-C₄alcynyle, C₁-C₃alcoxy, C₁-C₃halogénoalkyle, C₁-C₃halogénoalcoxy, NR⁶R⁷, et C₁-C₃thioalkyle.

4. Composé de Formule (I) selon l'une quelconque des revendications précédentes, R² étant halogène, cyano, C₁-C₆alkyle, C₁-C₆halogénoalkyle, C(O)OC₁-C₆alkyle, phényle, et benzyloxy.

5. Composé de Formule (I) selon l'une quelconque des revendications précédentes, X² étant O.

6. Composé de Formule (I) selon la revendication 5, R¹² étant choisi dans le groupe constitué par : hydrogène, C₁-C₆alkyle, CᵣalcoxyCₛalkyle, C₁-C₆halogénoalkyle, CᵣalcoxyCₛhalogénoalkyle, CᵣalkylthioCₛalkyle, C₂-C₆alcényle, C₂-C₆alcynyle, et -(CR^{a}R^{b})_{q}R¹¹.

7. Composé selon la revendication 5, R⁴ et R¹² conjointement avec les atomes auxquels ils sont joints, formant un système cyclique à 5 à 7 chaînons contenant éventuellement 1 ou 2 hétéroatomes supplémentaires indépendamment choisis parmi S sous la forme de S(O)ₚ, O et N.

8. Composé de Formule (I) selon l'une quelconque des revendications 1 à 4, X² étant NR¹⁰.

9. Composé de Formule (I) selon la revendication 8, R¹⁰ étant hydrogène ou C₁-C₆alkyle.

10. Composé de Formule (I) selon la revendication 8, R¹⁰ conjointement avec R⁴ et les atomes auxquels R¹⁰ et R⁴ sont joints formant un système cyclique à 5 à 7 chaînons contenant éventuellement de 1 à 3 hétéroatomes supplémentaires indépendamment choisis parmi S sous la forme de S(O)ₚ, O et N.

11. Composé de Formule (I) selon la revendication 8, R¹⁰ conjointement avec R¹² et l'atome d'azote auquel R¹⁰ et R¹² sont joints formant un système cyclique à 5 à 7 chaînons contenant éventuellement de 1 à 3 hétéroatomes supplémentaires indépendamment choisis parmi S sous la forme de S(O)ₚ, O et N.

12. Composé selon la revendication 8, R¹⁰ étant hydrogène ou C₁-C₆alkyle, et R¹² étant C₁-C₄alkyle, C₁-C₃alcoxy, -(CH₂)₃SCH₃, C₁-C₃halogénoalkyle, C₃-C₆alcynyle, ou (CR^{a}R^{b})_{q}R¹¹.

13. Composé de Formule (I) selon l'une quelconque des revendications 1 à 6, 8, 9, 11 et 12, R⁴ étant choisi dans le groupe constitué par hydrogène, méthyle, éthyle, allyle, but-2-ynyle, C(O)R⁹ et - (CH₂)_{q}R⁵.

14. Composé de Formule (I) selon la revendication 13, R⁹ étant C₁-C₆alcoxy.

15. Composition herbicide comprenant un composé selon l'une quelconque des revendications précédentes et un adjuvant de formulation acceptable sur le plan agricole.

16. Composition herbicide selon la revendication 15, comprenant en outre au moins un pesticide supplémentaire.

17. Composition herbicide selon la revendication 16, le pesticide supplémentaire étant un herbicide ou un phytoprotecteur herbicide.

18. Procédé de lutte contre des mauvaises herbes au niveau d'un site comprenant l'application au site d'une quantité, pour la lutte contre les mauvaises herbes, d'une composition selon l'une quelconque des revendications 15 à 17.

19. Utilisation d'un composé de Formule (I) tel que défini dans l'une quelconque des revendications 1 à 14, en tant qu'herbicide.
